# EUROPEAN PATENT SPECIFICATION

(11) **EP 4 580 553 B1**
(45) Date of publication and mention of the grant of the patent: **20.05.2026**
(21) Application number: 23806406.7
(22) Date of filing: 31.08.2023
(51) Int. Cl.: A61F 2/24

(54) **ASSEMBLIES AND METHODS FOR ATTACHING PROSTHETIC VALVE COMPONENTS TO A FRAME OF A PROSTHETIC HEART VALVE**
ANORDNUNGEN UND VERFAHREN ZUR BEFESTIGUNG VON HERZKLAPPENPROTHESENKOMPONENTEN AN EINEM RAHMEN EINER HERZKLAPPENPROTHESE
ENSEMBLES ET PROCÉDÉS DE FIXATION DE COMPOSANTS DE PROTHÈSE VALVULAIRE À UNE STRUCTURE D'UNE PROTHÈSE VALVULAIRE CARDIAQUE

(30) Priority: 31.08.2022 US 202263402808 P; 07.03.2023 US 202363450544 P
(43) Date of publication of application: 09.07.2025
(73) Proprietor: Edwards Lifesciences Corporation, Irvine, CA 92614 (US)
(72) Inventor: LEVI, Tamir S., 3079892 Caesarea (IL); SHERMAN, Elena, 3079892 Caesarea (IL); DALBOW, Brendan Michael, Irvine, CA 92614 (US); PHAM, Bich Hoang, Irvine, CA 92614 (US)
(74) Representative: Eisenführ Speiser
(86) International application number: PCT/US2023/031602
(87) International publication number: WO 2024/049943

(56) References cited:
- WO-A1-2022/011209
- US-A1- 2016 213 467

## Description

### CROSS-REFERENCE TO RELATED APPLICATIONS

This application claims the benefit of U.S. Provisional Patent Application Nos. 63/402,808, filed August 31, 2022, and 63/450,544, filed March 7, 2023.

### FIELD

The present disclosure relates to prosthetic heart valves, and in particular to outer coverings or skirts and/or leaflets for prosthetic heart valves and methods for attaching an outer skirt and/or leaflets to a frame of a prosthetic heart valve.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimallyinvasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

Most expandable, prosthetic heart valves comprise a cylindrical metal frame or stent and prosthetic leaflets mounted inside the frame. These valves can also include an outer covering or skirt disposed around an outer surface of the frame. The outer skirt can be configured to establish a seal with the native tissue when the prosthetic heart valve is placed at the implantation site (and thus may be referred to as sealing members).
US 2016/213467 A1 discloses a prosthetic heart valve that includes at least two leaflets being secured together along aligned edges thereof by a stitch of a single thread. The stitch includes at least one loop extending through a first aperture, around the aligned edges, and back through the first aperture.
WO 2022/011209 Al discloses prosthetic heart valves and methods of attaching soft components, such as leaflets or skirts, to frames of such prosthetic valves.

### SUMMARY

The invention is a method of making a prosthetic heart valve as defined by claims 1 and 12 and a prosthetic heart valve as defined in claim 7. Described herein are prosthetic heart valves, delivery apparatuses, and methods for implanting prosthetic heart valves. In particular, described herein are examples of outer skirts for prosthetic heart valves and methods of attaching such outer skirts to a frame of a prosthetic heart valve. Prosthetic heart valves can include a frame and a leaflet assembly arranged on an inside of the frame. The prosthetic heart valve can include an outer skirt arranged around a circumference of the frame and on an outer surface of the frame. The outer skirt can include an edge (a circumferentially extending edge) that is disposed at a level of the leaflets, and thus can contact the leaflets. In some examples, the edge (which may be an outflow edge of the outer skirt that has a rougher surface relative to other portions of the outer skirt) can be secured to the frame using an overcast or looped stitch pattern sewn along the edge or edge portion of the outer skirt. As one example, the stitch pattern along an edge portion of the outer skirt can comprise a plurality of stitches forming loops over the free edge of the edge portion that are connected by an in-and-out stitch line along the outer skirt that is distanced from the free edge. Whip stitches can then extend through the loops and around struts of the frame in order to attach the free edge of the outer skirt to the frame. Such attachment methods can position the free edge of the outer skirt on an outer surface of the frame struts, away from the leaflets, as well as distributing pulling forces on the outer skirt from the whip stitches away from the free edge. In some examples, a similar stitch pattern can be used to attach cusp edges of the leaflets to a frame or skirt of a prosthetic heart valve, thereby resulting in better flow patterns across the inflow edges of the leaflets during operation of the prosthetic valve. As such, the devices and methods disclosed herein can, among other things, overcome one or more of the deficiencies of typical prosthetic heart valves.

A method can comprise forming a stitching line along an edge portion of a first component for a prosthetic heart valve with a plurality of in-and-out stitches that extend along the edge portion and a plurality of loops that are spaced apart from one another in a direction of the in-and-out stitches. Each loop circles around an outer edge of the edge portion and extends between the edge and an intersection of two adjacent in-and-out stitches. The method further comprises attaching the edge portion to a second component of the prosthetic heart valve by extending a plurality of whip stitches through the plurality of loops and around or through the second component.

In some examples, the first component is an outer skirt, and the second component is a frame of the prosthetic heart valve.

In some examples, the first component is an outer skirt, and the second component is a fabric that is configured to be secured to a frame of the prosthetic heart valve.

In some examples, the first component is a cusp edge portion of a leaflet of a valvular assembly of the prosthetic heart valve, and the second component is a fabric component that is configured to be secured on an inside of a frame of the prosthetic heart valve.

In some examples, the first component is a cusp edge portion of a leaflet of a valvular assembly of the prosthetic heart valve, and the second component is struts of a frame of the prosthetic heart valve.

In some examples, the first component is a cusp edge portion of a leaflet of a valvular assembly of the prosthetic heart valve, and the second component is a suture directly wrapped around struts of a frame of the prosthetic heart valve.

In some examples, a method can comprise forming a stitching line along an edge portion of an outer skirt or a cusp edge of a leaflet for a prosthetic heart valve with a plurality of in-and-out stitches that extend along the edge portion and a plurality of loops that are spaced apart from one another in a direction of the in-and-out stitches. Each loop circles around a free edge of the edge portion, and the plurality of loops spaces the plurality of in-and-out stitches away from the free edge. The method further comprises attaching the edge portion to an additional component of the prosthetic heart valve by extending a plurality of whip stitches through the plurality of loops and around the additional component. The additional component can be struts of a circumferentially extending row of struts of an annular frame of the prosthetic heart valve, one or more sutures extending around the struts, or a fabric (such as a skirt or connecting component) that is configured to be secured to the frame.

In some examples, a method comprises forming a stitching line along an edge portion of an outer skirt for a prosthetic heart valve with a plurality of in-and-out stitches that extend along the edge portion and a plurality of loops that are spaced apart from one another in a direction of the in-and-out stitches. Each loop circles around a free edge of the edge portion, and the plurality of loops spaces the plurality of in-and-out stitches away from the free edge. The method further comprises attaching the edge portion to struts of a circumferentially extending row of struts of an annular frame of the prosthetic heart valve by extending a plurality of whip stitches through the plurality of loops and around the struts such that the free edge of the edge portion is positioned against radially outward facing surfaces of the struts and away from leaflets arranged on an inside of the annular frame.

In some examples, a method comprises forming a stitching line along an edge portion of an outer skirt for a prosthetic heart valve with a plurality of in-and-out stitches that extend along the edge portion, spaced away from a free edge of the edge portion, and a plurality of loops that are spaced apart from one another in a direction of the in-and-out stitches. Each loop circles around the free edge of the edge portion and extends between the edge and an intersection of two adjacent in-and-out stitches. The method further comprises attaching the edge portion to struts of a circumferentially extending row of struts of an annular frame of the prosthetic heart valve by extending a plurality of whip stitches through the plurality of loops and around the struts such that the free edge of the edge portion is positioned against radially outward facing surfaces of the struts and away from leaflets arranged on an inside of the annular frame.

In some examples, a method comprises forming a stitching line along an edge portion of an outer skirt for a prosthetic heart valve with a plurality of in-and-out stitches that extend along the edge portion, spaced away from a free edge of the edge portion, and a plurality of pairs of loops that are spaced apart from one another in a direction of the in-and-out stitches. Each pair of loops includes a first loop and a second loop, the first loop circling around the free edge of the edge portion and extending between the free edge and the second loop, and the second loop extending between the first loop and an intersection of two adjacent in-and-out stitches of the plurality of in-and-out stitches. The method further comprises attaching the edge portion to struts of a circumferentially extending row of struts of an annular frame of the prosthetic heart valve by extending whip stitches through the first loop of each pair of loops and around the struts such that the free edge of the edge portion is positioned against radially outward facing surfaces of the struts and away from leaflets arranged on an inside of the annular frame.

In some examples, a method comprises forming a stitching line along a cusp edge portion of a leaflet for a prosthetic heart valve with a plurality of in-and-out stitches that extend along the cusp edge portion, spaced away from an outermost cusp edge of the cusp edge portion, and a plurality of loops that are spaced apart from one another in a direction of the in-and-out stitches, where each loop circles around the cusp edge of the cusp edge portion and extends between the cusp edge and an intersection of two adjacent in-and-out stitches. The method further comprises attaching the cusp edge portion to a target component of the prosthetic heart valve by extending a plurality of whip stitches through the plurality of loops and around or through the target component such that the cusp edge of the cusp edge portion is tucked against the target component.

In some examples, a method comprises forming a stitching line along an edge portion of a first component for a prosthetic heart valve with a plurality of in-and-out stitches that extend along the edge portion, spaced away from an outermost edge of the edge portion, and a plurality of loops that are spaced apart from one another in a direction of the in-and-out stitches, where each loop circles around the edge of the edge portion and extends between the edge and an intersection of two adjacent in-and-out stitches. The method further comprises attaching the edge portion to a second component of the prosthetic heart valve by extending a plurality of whip stitches through the plurality of loops and around or through the second component such that the edge of the edge portion is coupled to and tucked against the second component.

In some examples, a method comprises one or more of the features recited in Examples 1-56, 94-107, 128-136, and 145 below.

A prosthetic heart valve can comprise a frame and a valve structure coupled to the frame. In addition to these components, a prosthetic heart valve can further comprise one or more of the components disclosed herein.

In some examples, a prosthetic heart valve can comprise a sealing member configured to reduce paravalvular leakage.

In some examples, the sealing member can be an outer sealing member disposed around an outer surface of the frame.

In some examples, the outer sealing member can comprise an outflow edge portion with a free outflow edge, the outflow edge portion including a stitching line comprising a plurality of in-and-out stitches extending along the outflow edge portion in parallel to but spaced away from the outflow edge, and a plurality of looped stitches that are spaced apart from one another in a direction of the plurality of in-and-out stitches. Each looped stitch loops around the outflow edge and extends between the outflow edge and an intersection of a corresponding two adjacent in-and-out stitches of the plurality of in-and-out stitches.

In some examples, the outflow edge portion can be attached to struts of the frame by additional stitches that extend through the plurality of looped stitches.

In some examples, a prosthetic heart valve can comprise a frame, a valve structure coupled to the frame, and an outer sealing member disposed around an outer surface of the frame. The outer sealing member can comprise an outflow edge portion with a free outflow edge, the outflow edge portion extending circumferentially around the frame and including a stitching line comprising a plurality of in-and-out stitches extending along the outflow edge portion in parallel to but spaced away from the outflow edge, and a plurality of looped stitches that are spaced apart from one another in a direction of the plurality of in-and-out stitches. Each looped stitch loops around the outflow edge and extends between the outflow edge and an intersection of a corresponding two adjacent in-and-out stitches of the plurality of in-and-out stitches. The outflow edge portion is attached to struts of the frame by additional stitches that extend through the plurality of looped stitches.

In some examples, a prosthetic heart valve can comprise a frame and a valve structure coupled to the frame, the valve structure comprising a plurality of leaflets, where a cusp edge portion of each leaflet is attached to a portion of angled struts of the frame or an inner skirt configured to be attached on an interior of the frame by a stitching line formed along the cusp edge portion, the stitching line comprising: a plurality of in-and-out stitches extending along the cusp edge portion in parallel to but spaced away from a free cusp edge of the cusp edge portion; and a plurality of looped stitches that are spaced apart from one another in a direction of the plurality of in-and-out stitches. Each looped stitch loops around the cusp edge and extends between the cusp edge and an intersection of a corresponding two adjacent in-and-out stitches of the plurality of in-and-out stitches. The prosthetic heart valve further comprises a plurality of stitches extending through the plurality of looped stitches and around the portion of angled struts, around one or more sutures extending around the portion of angled struts, or through the inner skirt such that the cusp edge is coupled to the portion of angled struts and/or the inner skirt.

In some examples, a prosthetic heart valve comprises an annular frame comprising a plurality of interconnected struts arranged into a plurality of circumferentially extending rows of struts including a first circumferentially extending row of struts that are disposed closer to an outflow end than an inflow end of the frame. The prosthetic heart valve further comprises an outer skirt disposed around an outer surface of the frame and comprising an outflow edge portion with a free outflow edge. The outflow edge portion extends circumferentially around the frame and includes a stitching line comprising a plurality of in-and-out stitches extending along the outflow edge portion in parallel to but spaced away from the outflow edge, and a plurality of looped stitches that are spaced apart from one another in a direction of the plurality of in-and-out stitches. Each looped stitch loops around the outflow edge and extends between the outflow edge and an intersection of a corresponding two adjacent in-and-out stitches of the plurality of in-and-out stitches. The prosthetic heart valve further comprises a plurality of whip stitches extending through the plurality of looped stitches and around struts of the first circumferentially extending row of struts such that the outflow edge is attached to the struts, against radially outward facing surfaces of the struts.

In some examples, a prosthetic heart valve comprises an annular frame comprising a plurality of interconnected struts arranged into a plurality of circumferentially extending rows of struts between an inflow end and an outflow end of the frame, where the plurality of circumferentially extending rows of struts includes a first circumferentially extending row of struts. The prosthetic heart valve further comprises an outer skirt disposed around an outer surface of the frame and comprising an edge portion with a free edge, where the edge portion extends circumferentially around the frame and includes a stitching line comprising: a plurality of in-and-out stitches extending along the edge portion in parallel to but spaced away from the free edge, and a plurality of pairs of looped stitches that are spaced apart from one another in a direction of the plurality of in-and-out stitches. Each pair of looped stitches includes a first looped stitch and a second looped stitch, where the first looped stitch loops around the free edge and extends between the free edge and the second loop, and where the second loop extends between the first loop and an intersection of a corresponding two adjacent in-and-out stitches of the plurality of in-and-out stitches. The prosthetic heart valve further comprises a plurality of whip stitches extending through the first looped stitch of each pair of looped stitches and around struts of the first circumferentially extending row of struts such that the free edge is attached to the struts, against radially outward facing surfaces of the struts.

In some examples, a prosthetic heart valve comprises an annular frame comprising a plurality of interconnected struts arranged into a plurality of circumferentially extending rows of angled struts between an inflow end and an outflow end of the frame, a plurality of leaflets disposed on an interior of the frame, where each leaflet comprises a cusp edge portion extending between opposing side portions disposed on opposite ends of an outflow edge of the leaflet, and wherein the cusp edge portion is attached to a portion of angled struts of the plurality of circumferentially extending rows of angled struts by a stitching line formed along the cusp edge portion. The stitching line comprises a plurality of in-and-out stitches extending along the cusp edge portion in parallel to but spaced away from a free cusp edge of the cusp edge portion, and a plurality of looped stitches that are spaced apart from one another in a direction of the plurality of in-and-out stitches, where each looped stitch loops around the cusp edge and extends between the cusp edge and an intersection of a corresponding two adjacent in-and-out stitches of the plurality of in-and-out stitches. The prosthetic heart valve further comprises a plurality of whip stitches extending through the plurality of looped stitches and around the portion of angled struts or around one or more sutures extending around the portion of angled struts such that the cusp edge is coupled to and tucked against the portion of angled struts.

In some examples, a prosthetic heart valve comprises an annular frame comprising a plurality of interconnected struts arranged into a plurality of circumferentially extending rows of angled struts between an inflow end and an outflow end of the frame, an inner skirt configured to be secured to a portion of angled struts of the plurality of circumferentially extending rows of angled struts, on an inside of the frame, and a plurality of leaflets disposed on an interior of the frame, where each leaflet comprises a cusp edge portion extending between opposing side portions disposed on opposite ends of an outflow edge of the leaflet, and wherein the cusp edge portion is attached to the inner skirt by a stitching line formed along the cusp edge portion. The stitching line comprises a plurality of in-and-out stitches extending along the cusp edge portion in parallel to but spaced away from a free cusp edge of the cusp edge portion, and a plurality of looped stitches that are spaced apart from one another in a direction of the plurality of in-and-out stitches, where each looped stitch loops around the cusp edge and extends between the cusp edge and an intersection of a corresponding two adjacent in-and-out stitches of the plurality of in-and-out stitches. The prosthetic heart valve further comprises a plurality of whip stitches extending through the plurality of looped stitches and through the inner skirt such that the cusp edge is coupled to and tucked against the inner skirt.

In some examples, a prosthetic heart valve comprises a frame comprising a plurality of interconnected struts, a plurality of leaflets disposed within the frame and configured to regulate a flow of blood through the frame in one direction, each leaflet comprising a cusp edge portion, and at least one suture extending along a cusp edge portion of at least a first leaflet of the plurality of leaflets, a plurality of in-and-out stitches extending through the suture and the cusp edge portion of the first leaflet, and a plurality of first stitches that extend around selected struts of the plurality of the interconnected struts and a portion of the in-and-out stitches to couple the cusp edge portion of the first leaflet to the frame.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a side view of a prosthetic heart valve, according to one example.
FIG. 2 is a side view of a frame of the prosthetic heart valve of FIG. 1.
FIG. 3 is a side view of a portion of the frame of FIG. 2, showing the portion of the frame in a straightened (non-annular) state.
FIG. 4 is a side view of an exemplary delivery apparatus configured to deliver and implant a radially expandable prosthetic heart valve at an implantation site.
FIG. 5 is a side view of an outer skirt for a prosthetic heart valve, according to an example, the outer skirt shown in a flattened configuration.
FIG. 6 is a schematic of a portion of an outer skirt and an exemplary stitching line along an edge portion of the outer skirt, the stitching line comprising in-and-out stitches that are spaced away from a free edge of the edge portion by a plurality of spaced apart single loops of the stitching line.
FIG. 7A is a schematic of the outer skirt and stitching line of FIG. 6 showing the edge portion secured to a strut of a frame of a prosthetic device using whip stitches that extend around the strut and through the loops of the stitching line.
FIG. 7B is a schematic cross-section showing the edge portion of the outer skirt of FIG. 7A secured to the strut of the frame using the whip stitches that extend around the strut and through the loops of the stitching line of FIG. 6.
FIG. 8A is a schematic of a portion of an outer skirt and an exemplary stitching line along an edge portion of the outer skirt, the stitching line comprising in-and-out stitches that are spaced away from a free edge of the edge portion by a plurality of spaced apart pairs of loops of the stitching line.
FIG. 8B is a schematic of the outer skirt and stitching line of FIG. 8A showing a second pass of alternating in-and-out stitches in the stitching line.
FIG. 9A shows an example of the stitching line of FIG. 8A applied to an undulating edge portion of the outer skirt with a first pass of in-and-out stitches.
FIG. 9B shows the example of the stitching line of FIG. 9A including a second pass of in-and-out stitches.
FIG. 10A shows an exterior view of a portion of a prosthetic valve frame, with the outer skirt with the stitching line of FIGS. 9A and 9B arranged around an outer surface of the frame and being secured to the struts of the frame with whip stitches that extend around the struts and through loops of the stitching line.
FIG. 10B shows an interior view of the frame and outer skirt of FIG. 10A, with the edge portion of the outer skirt attached to the frame struts with whip stitches extending through loops of the stitching line.
FIG. 11 is a schematic of the outer skirt and stitching line of FIG. 8A showing the edge portion secured to a strut of a frame of a prosthetic device using whip stitches that extend around the strut and double loop around the loops of the stitching line.
FIGS. 12A-12G show an exemplary process for forming the whip stitches of FIG. 11 such that they extend through the loops of the stitching line in a double-loop and around frame struts in order to secure the edge portion of the outer skirt to the prosthetic valve frame.
FIG. 13 is a perspective view of an outer skirt attached to a prosthetic valve frame using the stitching line and whip stitches of FIG. 11, according to the process presented in FIGS. 11 and 12A-12G.
FIG. 14 is a schematic of a leaflet for a prosthetic heart valve, an exemplary stitching line along a cusp edge portion of the leaflet, the stitching line comprising in-and-out stitches that are spaced away from a cusp edge of the cusp edge portion by a plurality of spaced apart single loops of the stitching line, and a plurality of whip stitches connecting the stitching line to an additional component for a prosthetic heart valve.
FIG. 15 is a detail view of a portion of the cusp edge portion, stitching line, and additional component of FIG. 14.
FIG. 16 is a plan view of an exemplary leaflet with the stitching line of FIG. 14 formed along the cusp edge portion of the leaflet and the plurality of in-and-out stitches extending through a suture extending along the cusp edge portion of the leaflet.
FIG. 17 is a flattened view of a leaflet assembly including a plurality of the leaflets of FIG. 16 secured together, wherein the stitching line of FIG. 16 is formed along the cusp edge portion of each leaflet.
FIG. 18 is a perspective view of frame of a prosthetic heart valve with the leaflet assembly of FIG. 17 arranged on an interior of the frame with commissures of the leaflet assembly coupled to the frame.
FIG. 19 is a detail view of an inflow end of the frame with cusp edge portions of the leaflet assembly of FIG. 17 coupled to struts of the frame using the stitching lines along the cusp edge portions.
FIG. 20 is another detail view of the inflow end of the frame showing the cusp edge portions of the leaflet assembly of FIG. 17 coupled to struts of the frame using the stitching lines along the cusp edge portions.
FIG. 21 is another detail view of the inflow end of the frame showing the leaflet assembly of FIG. 17 attached to the frame and an outer skirt disposed around an outer surface of the frame.
FIG. 22 is a plan view of a leaflet assembly in a flat configuration with leaflets of the leaflet assembly secured together with a connecting suture line disposed along a cusp edge portion of each of the leaflets.
FIG. 23 is a plan view of one leaflet with a connecting suture line secured to a cusp edge portion of the leaflet.
FIG. 24 is a schematic cross-sectional view of a portion of a prosthetic valve showing the leaflet of FIG. 23 secured to a strut of a frame and an outer skirt of the prosthetic heart valve with a stitch extending through the connecting suture line.
FIG. 25 is a perspective view of a portion of a prosthetic valve showing the leaflet of FIG. 23 secured to struts of a frame of the prosthetic heart valve with stitches extending around in-and-out stitches of the connecting suture line.
FIG. 26 is a perspective view of a portion of a prosthetic valve showing the leaflet of FIG. 23 secured to struts of a frame of the prosthetic heart valve with stitches extending around in-and-out stitches of the connecting suture line, around struts of the frame, and through an outer skirt disposed around an outer surface of the frame.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Overview of the Disclosed Technology

As introduced above, prosthetic heart valves can include a frame comprising interconnected struts, leaflets mounted inside the frame, and an outer skirt disposed around an outer surface of the frame. In some examples, an edge of the outer skirt that is disposed across the frame at a level of the leaflets can be jagged, rough, or otherwise abrasive (for example, due to exposed fibers that make up the skirt or from a molten edge formed by melting fibers along the edge to reduce fraying of the fabric of the outer skirt). As such, the edge of the outer skirt can contact the leaflets during operation of the prosthetic heart valve and reduce a longevity of the leaflets. In order to avoid the leaflets contacting the edge of the outer skirt during operation of the prosthetic heart valve, in some instances, the edge of the outer skirt can be folded outward, away from the frame and the leaflets. The folded edge can then be secured to the frame with stitches that extend through the skirt's folded edge and around struts of the frame. In some examples, the stitches can pass through the folded skirt layers close to the edge of the skirt, which may result in fraying or unraveling of the skirt's edge. Additionally, skirt attachment methods that include folding an edge of the skirt prior to stitching can increase assembly time of the prosthetic heart valve (since such processes cannot be easily automated).

In some examples, cusp edge potions (or scallop edges) of the leaflets can be sutured to the frame or alternate components (e.g., fabric skirts) in a way that creates a step-like edge that can create undesirable flow disturbances at these regions (for example, uneven flow or turbulent flow). Such flow disturbances may reduce an efficiency of the prosthetic heart valve.

Described herein are various methods for attaching (for example, stitching) an outer skirt to a frame of a prosthetic heart valve that result in an edge of the outer skirt (which may be positioned at a level of the leaflets) being positioned away from the leaflets while also transferring pulling forces on the skirt from stitches used to attach the outer skirt to the frame away from the skirt's edge. As a result, a durability and longevity of the prosthetic heart valve can be increased while also reducing a time and effort for assembling the prosthetic heart valve.

Also described herein are methods for attaching (for example, stitching) cusp edges of leaflets to a target component of a prosthetic heart valve (such as a fabric skirt, sutures extending around frame struts of the frame, or directly to the frame struts) that result in the cusp edges being tightly tucked to the target component. As a result, a more continuous or smooth geometry can be created at the inflow edges of the leaflets, thereby reducing a likelihood of blood flow disturbances along the leaflet inflow edges. Thus, an efficiency of the prosthetic heart valve can be increased.

Prosthetic valves disclosed herein can be radially compressible and expandable between a radially compressed state and a radially expanded state. Thus, the prosthetic valves can be crimped on or retained by an implant delivery apparatus in the radially compressed state while being advanced through a patient's vasculature on the delivery apparatus. The prosthetic valve can be expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

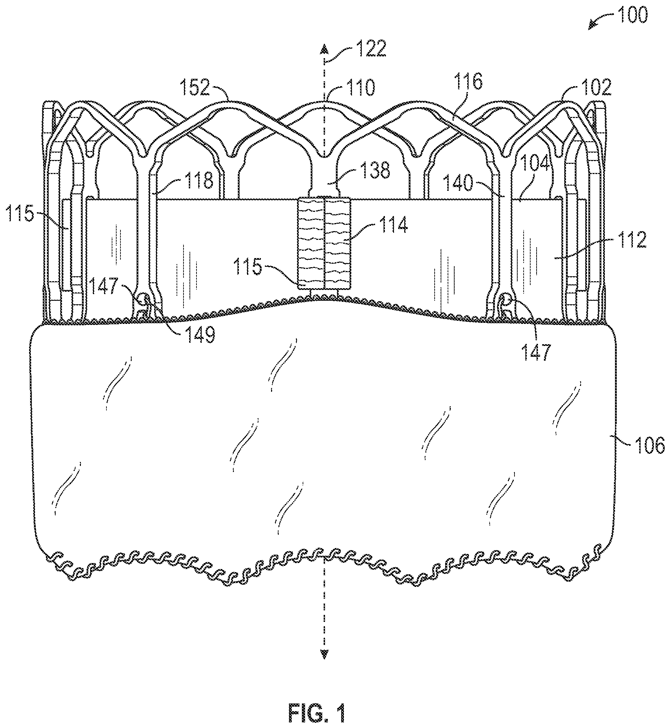

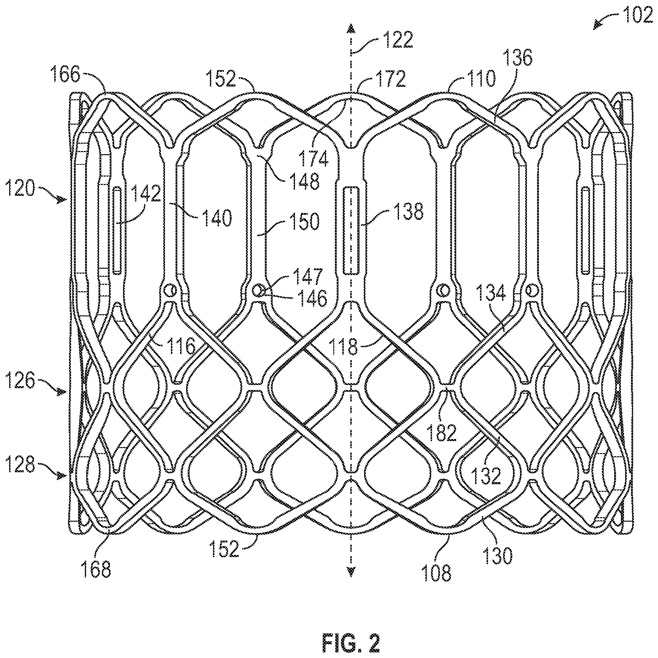

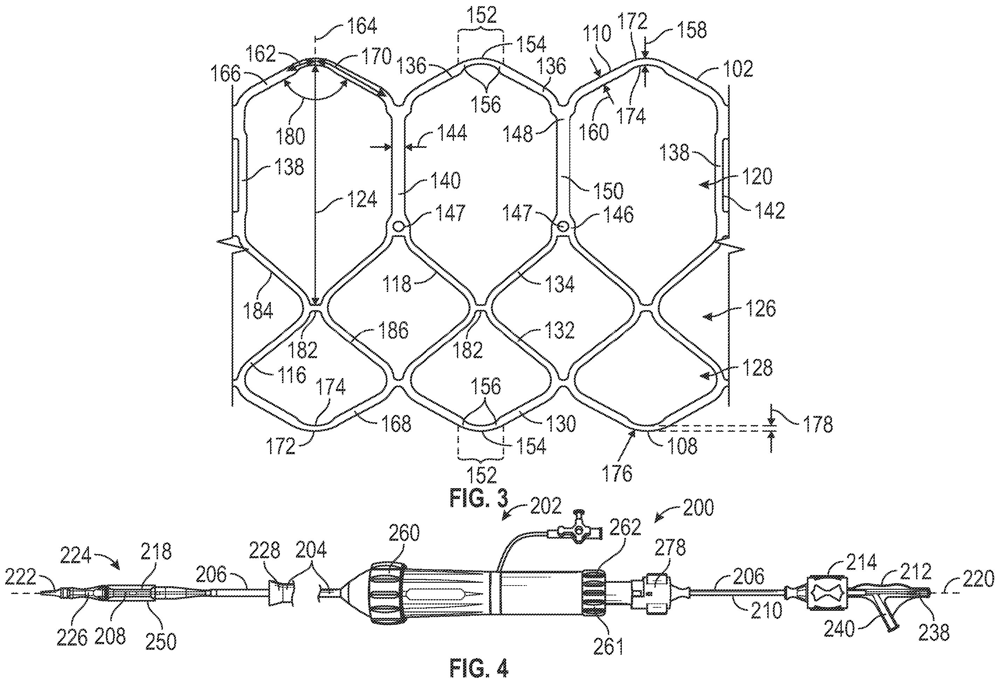

FIG. 1 illustrates an exemplary prosthetic device (e.g., prosthetic heart valve) comprising a frame, leaflets secured on an inside of the frame, and an outer skirt disposed around an outer surface of the frame. In some examples, the frame can comprise a plurality of interconnected and angled struts and apex regions that extend and/or curve between the angled struts at an inflow end and outflow end of the frame, as shown in FIGS. 2 and 3. The prosthetic device can be advanced through a patient's vasculature, such as to a native heart valve, by a delivery apparatus, such as the exemplary delivery apparatus shown in FIG. 4.

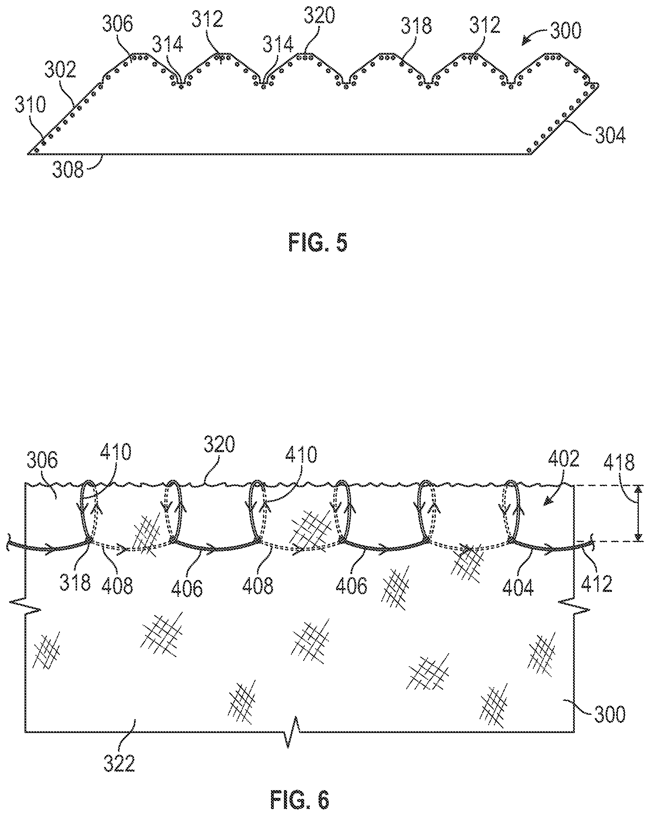

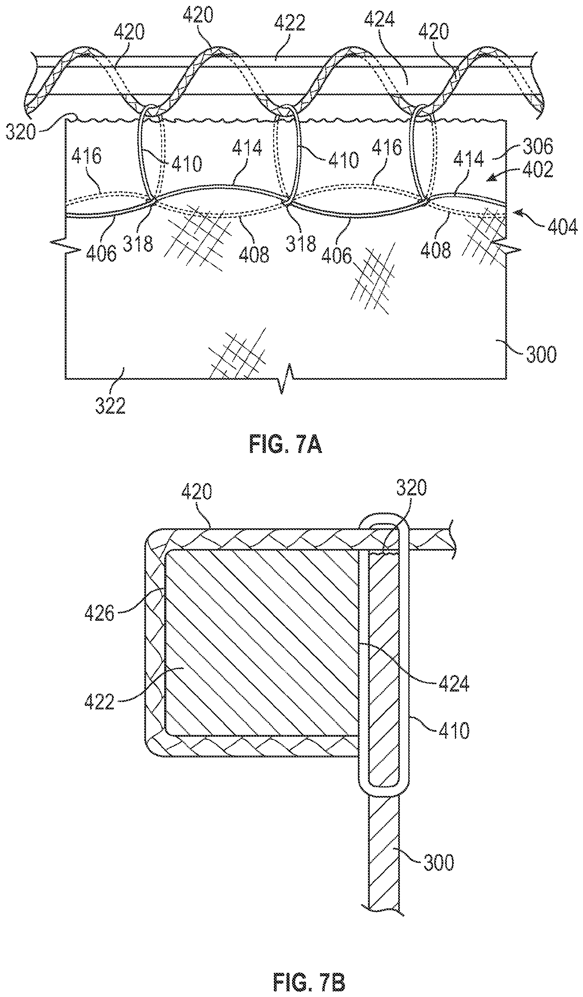

An exemplary outer skirt for a prosthetic heart valve is shown in FIG. 5. The outer skirt can comprise an edge, such as an outflow edge or edge portion, that is configured to be secured to struts of the frame. In some examples, the edge portion of the outer skirt can be secured to the frame using an overcast stitch pattern stitched along the edge portion of the outer skirt. As one example, the stitch pattern along the edge portion of the outer skirt can comprise a plurality of stitches forming loops over an outer or free edge of the edge portion that are connected by an in-and-out stitch line along the outer skirt that is spaced away from the free edge (FIG. 6). Whip stitches can then extend through the loops and around struts of the frame in order to attach the edge of the outer skirt to the frame (FIGS. 7A and 7B).

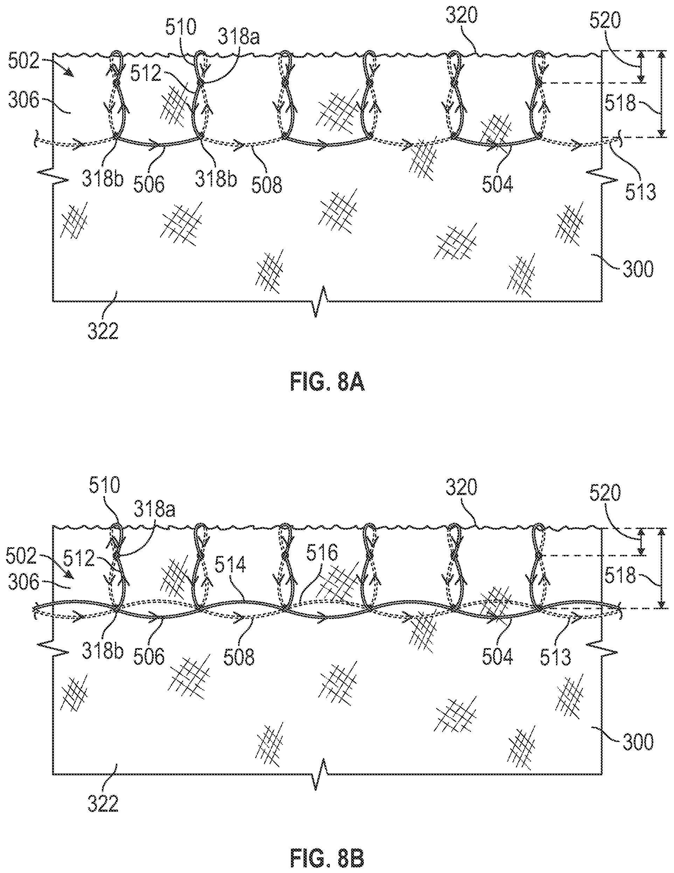

FIGS. 8A-13 show another example of a similar overcast stitch pattern used to attach the edge portion of the outer skirt to the frame, but the overcast stitch pattern comprises two loops between the free edge and the in-and-out stitch line in the outer skirt. This stitch pattern can better hold the loops of the overcast stitching line and the whip stitches extending through the second loop around the skirt edge in place.

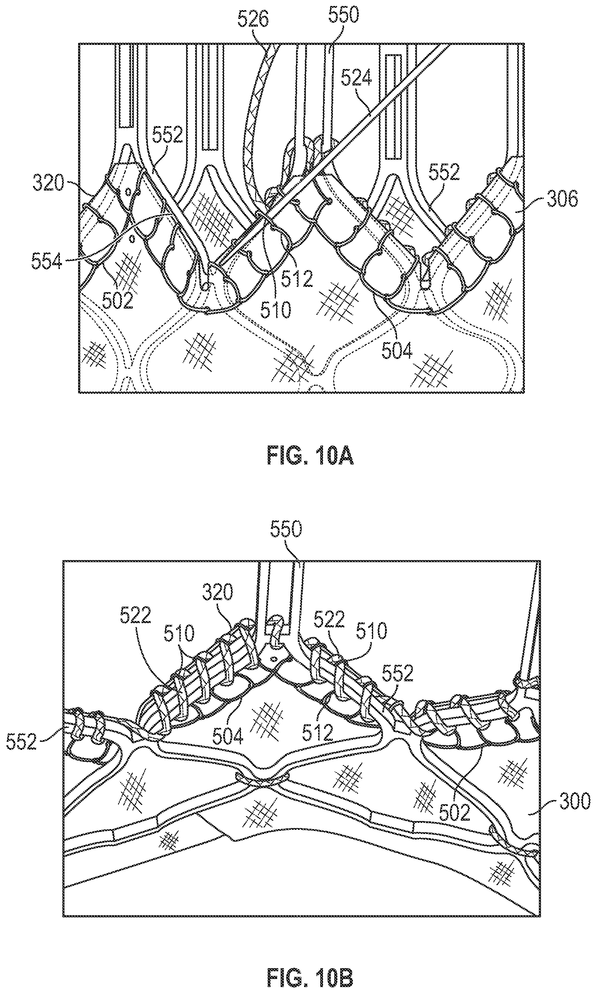

Such stitching patterns result in the edge (for example, a rougher edge) of the outer skirt to be positioned and held along a radially outward facing surface of the frame struts, thereby positioning the skirt edge away from the leaflets (as seen in FIGS. 10B, 12F, 12G, and 13).

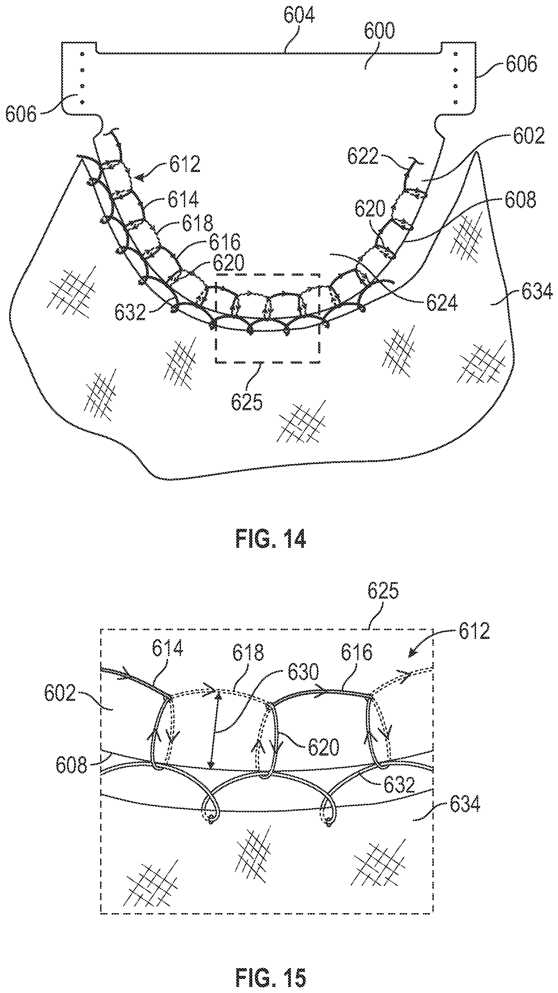

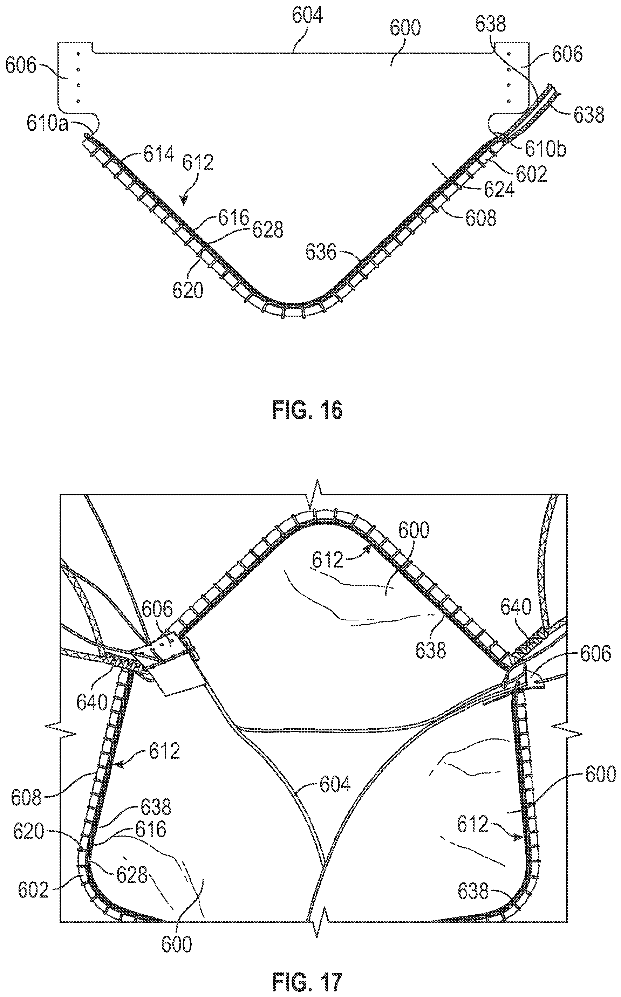

FIGS. 14-16 show an example of an overcast stitch pattern stitched along a cusp edge portion of a leaflet of a prosthetic heart valve, thereby resulting in an overcast stitch line comprising a plurality of in-and-out stitches and loops. The cusp edge portion can be attached to an additional component by a plurality of whip stitches that extend through the loops of the overcast stitch line and around or through the additional component. In some examples, as shown in FIGS. 17-21, the cusp edge portions of the leaflets can be secured to struts of the frame of the prosthetic valve using the overcast stitch line.

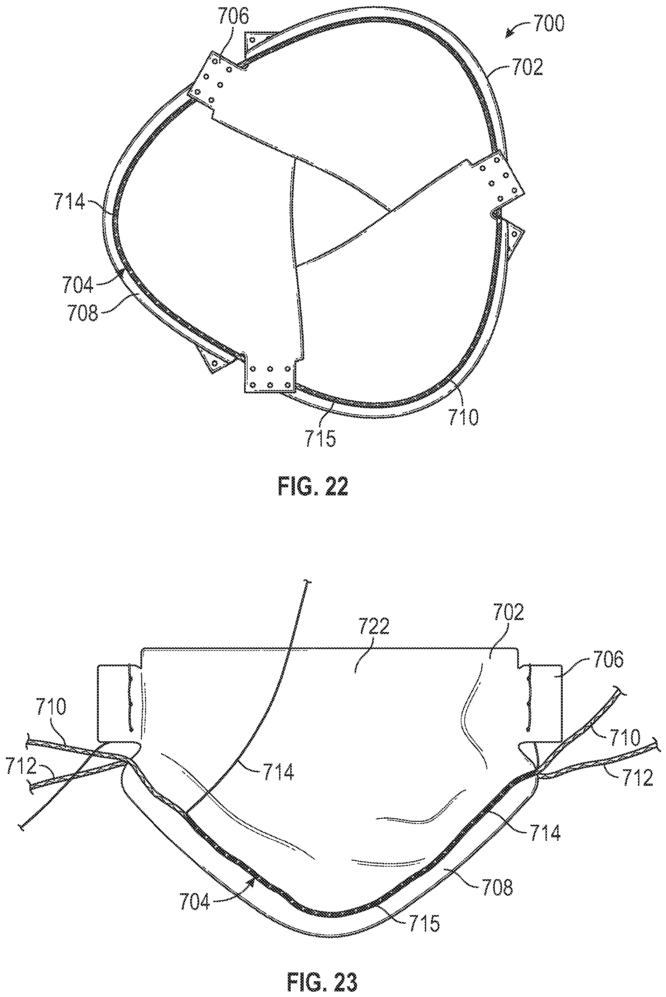

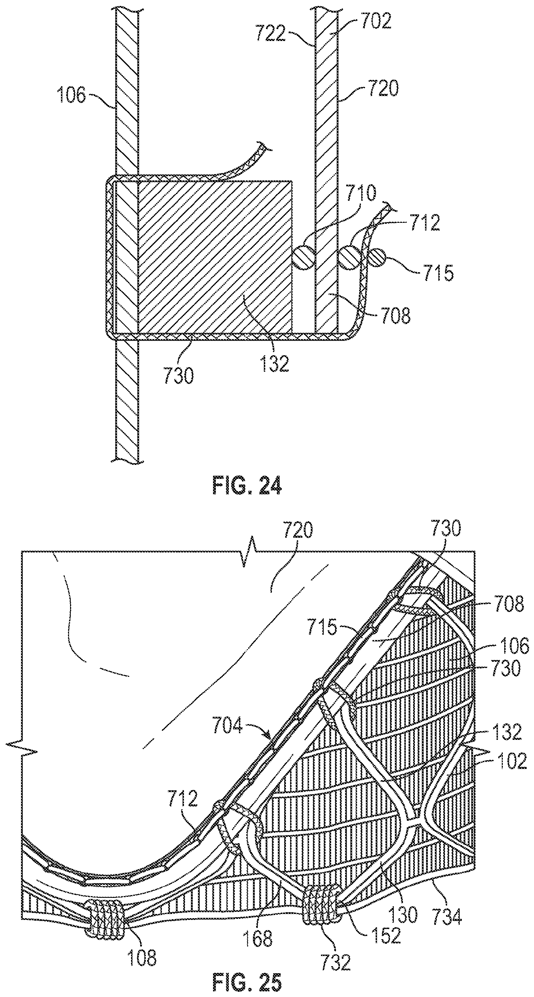

In some examples, as shown in FIGS. 22 and 23, the cusp edge portions of the leaflets can comprise in-and-out stitch lines. The cusp edge portions of the leaflets can then be secured to the frame by stitches extending through or around the in-and-out stitches of the in-and-out stitch lines and around struts of the frame, as shown in FIGS. 24-26.

### Examples of the Disclosed Technology

FIG. 1 shows a prosthetic heart valve 100 (prosthetic valve), according to one example. Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In another example, the disclosed prosthetic valves can be implanted within a docking device implanted within or at the native mitral valve, such as disclosed in PCT Publication No. WO2020/247907. In another example, the disclosed prosthetic valves can be implanted within a docking device implanted within the superior or inferior vena cava for replacing the function of a diseased tricuspid valve, such as disclosed in U.S. Publication No. 2019/0000615.

The prosthetic heart valve 100 can include a stent or frame 102, a valvular structure 104, and a perivalvular outer sealing member or outer skirt 106. The prosthetic heart valve 100 (and the frame 102) can have an inflow end 108 and an outflow end 110. The valvular structure 104 can be disposed on an interior of the frame 102 while the outer skirt 106 is disposed around an outer surface of the frame 102.

The valvular structure 104 can comprise a plurality of leaflets 112 (e.g., three leaflets, as shown in FIG. 1), collectively forming a leaflet structure, which can be arranged to collapse in a tricuspid arrangement. The leaflets 112 can be secured to one another at their adjacent sides (e.g., commissure tabs) to form commissures 114 of the valvular structure 104. For example, each leaflet 112 can comprise opposing commissure tabs disposed on opposite sides of the leaflet 112 and a cusp edge portion extending between the opposing commissure tabs. The cusp edge portion of the leaflets 112 can have an undulating, curved scalloped shape, and can be secured directly to the frame 102 (e.g., by sutures). However, in alternate examples, the cusp edge portion of the leaflets 112 can be secured to an inner skirt which is then secured to the frame 102. In some examples, the leaflets 112 can be formed of pericardial tissue (e.g., bovine pericardial tissue), biocompatible synthetic materials, or various other suitable natural or synthetic materials as known in the art and described in U.S. Patent No. 6,730,118.

In some examples, the outer skirt 106 can be an annular skirt. In some instances, the outer skirt 106 can comprise one or more skirt portions that are connected together and/or individually connected to the frame 102. The outer skirt 106 can comprise a fabric or polymeric material, such as ePTFE, PTFE, PET, TPU, UHMWPE, PEEK, PE, etc. In some instances, instead of having a relatively straight upper edge portion, as shown in FIG. 1, the outer skirt 106 can have an undulating upper edge portion that extends along and is secured to the angled struts 134. Examples of such outer skirts, as well as various other outer skirts, that can be used with the frame 102 can be found in U.S. provisional patent application No. 63/366,599 filed June 17, 2022.

The frame 102 can be radially compressible and expandable between a radially compressed (or collapsed) configuration and a radially expanded configuration (the expanded configuration is shown in FIG. 1). The frame 102 is shown alone in FIG. 2 and a portion of the frame 102 in a straightened (non-annular) configuration is shown in FIG. 3.

The frame 102 can be made of any of various suitable plastically-expandable materials (e.g., stainless steel, etc.) or self-expanding materials (e.g., Nitinol). When constructed of a plastically-expandable material, the frame 102 (and thus the valve 100) can be crimped to a radially compressed state on a delivery catheter and then expanded inside a patient by an inflatable balloon or equivalent expansion mechanism. When constructed of a self-expandable material, the frame 102 (and thus the valve 100) can be crimped to a radially compressed state and restrained in the compressed state by insertion into a sheath or equivalent mechanism of a delivery catheter. Once inside the body, the valve can be advanced from the delivery sheath, which allows the valve to expand to its functional size.

Suitable plastically-expandable materials that can be used to form the frames disclosed herein (e.g., frame 102) include metal alloys, polymers, or combinations thereof. Example metal alloys can comprise one or more of the following: nickel, cobalt, chromium, molybdenum, titanium, or other biocompatible metal. In some examples, the frame 102 can comprise stainless steel. In some examples, the frame 102 can comprise cobalt-chromium. In some examples, the frame 102 can comprise nickel-cobalt-chromium. In some examples, the frame 102 comprises a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight.

As shown in FIGS. 2 and 3, the frame 102 can comprise a plurality of interconnected struts 116 which form multiple rows of open cells 118 between the outflow end 110 and the inflow end 108 of the frame 102. In some examples, as shown in FIGS. 2 and 3, the frame 102 can comprise three rows of cells 118 with a first (upper in the orientation shown in FIGS. 2 and 3) row of cells 120 disposed at the outflow end 110. The first row of cells 120 comprises cells 118 that are elongated in an axial direction (relative to a central longitudinal axis 122 of the frame 102), as compared to cells 118 in the remaining rows of cells. For example, the cells 118 of the first row of cells 120 can have a longer axial length 124 (FIG. 3) than cells 118 in the remaining rows of cells, which can include a second row of cells 126 and a third row of cells 128, the third row of cells 128 disposed at the inflow end 108 and the second row of cells 126 disposed between the first row of cells 120 and the third row of cells 128.

In some examples, as shown in FIG. 2, each row of cells comprises nine cells 118. Thus, in such examples, the frame 102 can be referred to as a nine-cell frame.

In alternate examples, the frame 102 can comprise more than three rows of cells (e.g., four or five) and/or more or less than nine cells per row. In some examples, the cells 118 in the first row of cells 120 may not be elongated compared to cells 118 in the remaining rows of cells of the frame 102 (the second row of cells 126 and the third row of cells 128).

The interconnected struts 116 can include a plurality of angled struts 130, 132, 134, and 136 arranged in a plurality of rows of circumferentially extending rows of angled struts, with the rows being arrayed along the length of the frame 102 between the outflow end 110 and the inflow end 108. For example, the frame 102 can comprise a first row of angled struts 130 arranged end-to-end and extending circumferentially at the inflow end 108 of the frame; a second row of circumferentially extending, angled struts 132; a third row of circumferentially extending, angled struts 134; and a fourth row of circumferentially extending, angled struts 136 at the outflow end 110 of the frame 102. The fourth row of angled struts 136 can be connected to the third row of angled struts 134 by a plurality of axially extending window struts 138 (or window strut portions) and a plurality of axial (or axially extending) struts 140. The axially extending window struts 138 (which can also be referred to as axial struts that include a commissure window) define commissure windows (e.g., open windows) 142 that are spaced apart from one another around the frame 102, in a circumferential direction, and which are adapted to receive a pair of commissure tabs of a pair of adjacent leaflets 112 arranged into a commissure (e.g., commissure 114 shown in FIG. 1). In some examples, the commissure windows 142 and/or the axially extending window struts 138 defining the commissure windows 142 can be referred to herein as commissure features or commissure supports, each commissure feature or support configured to receive and/or be secured to a pair of commissure tabs of a pair of adjacent leaflets.

One or more (for example, two, as shown in FIGS. 2 and 3) axial struts 140 can be positioned between, in the circumferential direction, two commissure windows 142 formed by the window struts 138. Since the frame 102 can include fewer cells per row (e.g., nine) and fewer axial struts 140 between each commissure window 142, as compared to some more traditional prosthetic heart valves, each cell 118 can have an increased width (in the circumferential direction), thereby providing a larger opening for blood flow and/or coronary access.

Each axial strut 140 and each window strut 138 extends from a location defined by the convergence of the lower ends (e.g., ends arranged inward of and farthest away from the outflow end 110) of two angled struts 136 (which can also be referred to as an upper strut junction or upper elongated strut junction) to another location defined by the convergence of the upper ends (e.g., ends arranged closer to the outflow end 110) of two angled struts 134 (which can also be referred to as a lower strut junction or lower elongate strut junction). Each axial strut 140 and each window strut 138 forms an axial side of two adjacent cells of the first row of cells 120.

In some examples, as shown in FIG. 3, each axial strut 140 can have a width 144 (FIG. 3) that is larger than a width of the angled struts 130, 132, 134, and 136. As used herein, a "width" of a strut is measured between opposing locations on opposing surfaces of a strut that extend between the radially facing inner and outer surfaces of the strut (relative to the central longitudinal axis 122 of the frame 102). A "thickness" of a strut is measured between opposing locations on the radially facing inner and outer surfaces of a strut and is perpendicular to the width of the strut. In some examples, the width 144 of the axial struts 140 is 50-200%, 75-150%, or at least 100% larger than (e.g., double) the width of the angled struts of the frame 102.

By providing the axial struts 140 with the width 144 that is greater than the width of other, angled struts of the frame 102, a larger contact area is provided for when the leaflets 112 contact the wider axial struts 140 during systole, thereby distributing the stress and reducing the extent to which the leaflets 112 may fold over the axial struts 140, radially outward through the cells 118. As a result, a long-term durability of the leaflets 112 can be increased.

Since the cells 118 of the frame 102 can have a relatively large width compared to alternate prosthetic valves that have more than nine cells per row (as introduced above), the wider axial struts 140 can be more easily incorporated into the frame 102, without sacrificing open space for blood flow and/or coronary access.

Commissure tabs 115 of adjacent leaflets 112 can be secured together to form commissures 114 (FIG. 1). Each commissure 114 of the prosthetic heart valve 100 comprises two commissure tabs 115 paired together, one from each of two adjacent leaflets 112, and extending through a commissure window 142 of the frame 102. Each commissure 114 can be secured to the window struts 138 forming the commissure window 142.

The cusp edge portion (e.g., scallop edge) of each leaflet 112 can be secured to the frame 102 via one or more fasteners (e.g., sutures). In some examples, the cusp edge portion of each leaflet 112 can be secured directly to the struts of the frame 102 (e.g., angled struts 130, 132, and 134). For example, the cusp edge portions of the leaflets 112 can be sutured to the angled struts 130, 132, and 134 that generally follow the contour of the cusp edge portions of the leaflets 112.

In some examples, the cusp edge portion of the leaflets 112 can be secured to an inner skirt and the inner skirt can then be secured directly to the frame 102.

Various methods for securing the leaflets 112 to a frame, such as the frame 102, are disclosed in U.S. provisional patent applications 63/278,922, filed November 12, 2021, and 63/300,302, filed January 18, 2022.

As shown in FIGS. 2 and 3, in some examples, one or more of or each of the axial struts 140 can comprise an inflow end portion 146 (e.g., an end portion that is closest to the inflow end 108) and an outflow end portion 148 that are widened relative to a middle portion 150 of the axial strut 140 (which can be defined by the width 144). In some instances, the inflow end portion 146 of the axial strut 140 can comprise an aperture 147. The apertures 147 can be configured to receive fasteners (e.g., sutures) for attaching soft components of the prosthetic heart valve 100 to the frame 102. For example, in some instances, the outer skirt 106 can be positioned around the outer surface of the frame 102 and an upper or outflow edge portion of the outer skirt 106 can be secured to the apertures 147 by fasteners 149 (e.g., sutures), as shown in FIG. 1.

The interconnected struts 116 can also comprise horizontal struts 182 that extend between adjacent cells 118 of a row of cells of the frame 102 (FIGS. 2 and 3). The horizontal struts 182 can extend in a circumferential direction and also be referred to as circumferentially extending struts 182. The horizontal struts 182 can connect angled struts of two adjacent rows of angled struts of the frame 102 to one another. For example, each horizontal strut 182 can connect to two angled struts of one row of struts (for example, struts 134 shown in FIG. 3) and two angled struts in another, adjacent row of struts (for example, struts 132 shown in FIG. 3). As a result, an angled strut 184 extending between an axially extending window strut 138 and the horizontal strut 182 and an angled strut 186 extending between the horizontal strut 182 and another horizontal strut 182 disposed adjacent to the inflow end 108 of the frame 102 can be aligned along an angled line that can follow a scallop line of the leaflets (when the leaflets are attached to the frame 102). Thus, the horizontal struts 182 can allow the angled struts to follow a shape that more closely matches a shape of the scallop line of the leaflets when the frame 102 is in the radially expanded configuration (as shown in FIGS. 2 and 3). Additionally, the horizontal struts 182 can serve as spacers that can maintain a specified gap between the angled struts when the frame 102 is in the radially compressed configuration, thereby reducing a risk of pinching the leaflets between the struts in the radially compressed configuration.

The frame 102 can further comprise a plurality of apex regions 152 formed at the inflow end 108 and the outflow end 110, each apex region 152 extending and forming a junction between two angled struts 130 at the inflow end 108 or two angled struts 136 at the outflow end 110. As such, the apex regions 152 are spaced apart from one another, in a circumferential direction at the inflow end 108 and the outflow end 110.

Each apex region 152 can comprise an apex 154 (the highest or most outward extending, in an axial direction, point) and two thinned (or narrowed) strut portions 156, one thinned strut portion 156 extending from either side of the apex 154 to a corresponding, wider, angled strut 136 (at the outflow end 110) or angled strut 130 (at the inflow end 108) (FIG. 3). In this way, each of the apex regions 152 at the outflow end 110 can form a narrowed transition region between and relative to the two angled struts 136 extending from the corresponding apex region 152 and each of the apex regions 152 at the inflow end 108 can form a narrowed transition region between and relative to the two angled struts 130 extending from the corresponding apex region 152.

The thinned strut portions 156 of the apex regions 152 can have a width 158 that is smaller than a width 160 of the angled struts 130 or 136 (FIG. 3). In some examples, the width 158 can be a uniform width (e.g., along an entire length of the strut portion 156). In some examples, the width 158 of the thinned strut portions 156 can be from about 0.06 - 0.15 mm smaller than the width 160 of the angled struts 130 and/or 136.

The thinned strut portions 156 of the apex regions 152 can have a first length 162 (FIG. 3). In some examples, the first length 162 is in a range of 0.8-1.4 mm, 0.9-1.2 mm, 0.95-1.05 mm, or about 1.0 mm (e.g., ±0.03 mm). In alternate examples, the first length 162 is in a range of 0.3-0.7 mm, 0.4-0.6 mm, 0.45-0.55 mm, or about 0.5 mm (e.g., ±0.03 mm).

Thus, each outflow apex region 152 can include two thinned strut portions 156 having the first length 162, each extending from the apex 154, outward relative to a central longitudinal axis 164 of the cells 118. Thus, a total length of the apex region 152 can be two times the first length 162.

Each apex region 152 and two corresponding angled struts 136 at the outflow end 110 can form an outflow strut 166 and each apex region 152 and two corresponding angled struts 130 at the inflow end 108 can form an inflow strut 168.

Each outflow strut 166 and inflow strut 168 can have a length that includes an apex region 152 and the two angled struts 136 or 130 (or strut portions), respectively, on either side of the apex region 152. One half the total length of each outflow strut 166 and inflow strut 168 is shown in FIG. 3 as length 170, which extends from an end of one angled strut 136 or 130 to the central longitudinal axis 164. Thus, the length of each outflow strut 166 and inflow strut 168 is two times length 170. In some examples, the length 170 for half of each inflow strut 168 can be different than the length 170 for half of each outflow strut 166.

In some instances, the length of each thinned strut portion 156 can be at least 25% of the length 170 of the corresponding half outflow strut 166 or inflow strut 168. Said another way, the length of each apex region 152 (a total length being two times the first length 162) can be at least 25% of the total length (two times length 170) of the outflow strut 166 or inflow strut 168. In some examples, the length of each apex region 152 can be more than 25% of the total length of the corresponding outflow strut 166 or inflow strut 168, such as 25-35%.

In some examples, each apex region 152 can comprise a curved, axially facing outer surface 172 and an arcuate or curved, axially facing inner depression 174 which forms the thinned strut portions 156. For example, the curved inner depression 174 can depress toward the curved outer surface 172 from an inner surface of the angled strut portions 156, thereby forming the smaller width thinned strut portions 156. Thus, the curved inner depressions 174 can be formed on a cell side of the apex region 152 (e.g., as opposed to the outside of the apex region 152).

In some examples, the curved outer surface 172 of each apex region 152 can form a single, continuous curve from one angled strut portion 156 on a first side of the apex region 152 to another angled strut portion 156 on an opposite, second side of the apex region 152 (for example, the curved outer surface 172 can have a constant curvature).

Each apex region 152 can have a radius of curvature 176, along the curved outer surface 172 (e.g., in some instances, along an entirety or an entire length of the curved outer surface 172) (FIG. 3). In some instances, the radius of curvature 176 at the apex 154 and/or along the entire curved outer surface 172 of the apex region 152 can be greater than 1 mm. In some instances, the radius of curvature 176 can be in a range of 1-20 mm, 3-16 mm, or 8-14 mm. In some instances, the radius of curvature 176 can be greater than 10 mm. The radius of curvature 176 can be dependent on (and thus change due to changes in) the width 158 (e.g., the amount of reduction in width from the angled struts 130 or 136) and the first length 162 of the thinned strut portions 156.

Further, a height (an axial height) 178 of the apex regions 152, which can be defined in the axial direction from an outer surface of the two angled struts 130 or 136 to the curved outer surface 172 of the apex region 152 at the apex 154, can be the width 158 of the thinned strut portions 156 (FIG. 3). In this way, the height 178 of the apex regions 152 can be relatively small and not add much to the overall axial height of the radially expanded frame 102. Thus, the leaflets 112 secured to the frame 102 (FIG. 1) can be disposed close to the inflow end 108, thereby leaving a lar If ger open space at the outflow end 110 of the frame 102 that is not blocked by the leaflets 112.

In some examples, each of the apex region 152 can form an angle 180 between the two angled struts 130 or 136 extending from either side of the corresponding apex region 152 (FIG. 3). In some instances, the angle 180 can be in a range of 120 (not inclusive) to 140 degrees (e.g., such that the angle 180 is greater than 120 degrees and less than or equal to 140 degrees).

Additional details and examples of frames for prosthetic heart valves that include apex regions can be found in PCT Application No. PCT/US2022/025687.

FIG. 4 shows a delivery apparatus 200, according to an example, that can be used to implant an expandable prosthetic heart valve (e.g., the prosthetic heart valve 100 of FIG. 1 and/or any of the other prosthetic heart valves described herein). In some examples, the delivery apparatus 200 is specifically adapted for use in introducing a prosthetic valve into a heart.

The delivery apparatus 200 in the illustrated example of FIG. 4 is a balloon catheter comprising a handle 202 and a steerable, outer shaft 204 extending distally from the handle 202. The delivery apparatus 200 can further comprise an intermediate shaft 206 (which also may be referred to as a balloon shaft) that extends proximally from the handle 202 and distally from the handle 202, the portion extending distally from the handle 202 also extending coaxially through the outer shaft 204. Additionally, the delivery apparatus 200 can further comprise an inner shaft 208 extending distally from the handle 202 coaxially through the intermediate shaft 206 and the outer shaft 204 and proximally from the handle 202 coaxially through the intermediate shaft 206.

The outer shaft 204 and the intermediate shaft 206 can be configured to translate (e.g., move) longitudinally, along a central longitudinal axis 220 of the delivery apparatus 200, relative to one another to facilitate delivery and positioning of a prosthetic valve at an implantation site in a patient's body.

The intermediate shaft 206 can include a proximal end portion 210 that extends proximally from a proximal end of the handle 202, to an adaptor 212. A rotatable knob 214 can be mounted on the proximal end portion 210 and can be configured to rotate the intermediate shaft 206 around the central longitudinal axis 220 and relative to the outer shaft 204.

The adaptor 212 can include a first port 238 configured to receive a guidewire therethrough and a second port 240 configured to receive fluid (e.g., inflation fluid) from a fluid source. The second port 240 can be fluidly coupled to an inner lumen of the intermediate shaft 206.

The intermediate shaft 206 can further include a distal end portion that extends distally beyond a distal end of the outer shaft 204 when a distal end of the outer shaft 204 is positioned away from an inflatable balloon 218 of the delivery apparatus 200. A distal end portion of the inner shaft 208 can extend distally beyond the distal end portion of the intermediate shaft 206.

The balloon 218 can be coupled to the distal end portion of the intermediate shaft 206.

In some examples, a distal end of the balloon 218 can be coupled to a distal end of the delivery apparatus 200, such as to a nose cone 222 (as shown in FIG. 4), or to an alternate component at the distal end of the delivery apparatus 200 (e.g., a distal shoulder). An intermediate portion of the balloon 218 can overlay a valve mounting portion 224 of a distal end portion of the delivery apparatus 200 and a distal end portion of the balloon 218 can overly a distal shoulder 226 of the delivery apparatus 200. The valve mounting portion 224 and the intermediate portion of the balloon 218 can be configured to receive a prosthetic heart valve in a radially compressed state. For example, as shown schematically in FIG. 4, a prosthetic heart valve 250 (which can be one of the prosthetic valves described herein) can be mounted around the balloon 218, at the valve mounting portion 224 of the delivery apparatus 200.

The balloon shoulder assembly, including the distal shoulder 226, is configured to maintain the prosthetic heart valve 250 (or other medical device) at a fixed position on the balloon 218 during delivery through the patient's vasculature.

The outer shaft 204 can include a distal tip portion 228 mounted on its distal end. The outer shaft 204 and the intermediate shaft 206 can be translated axially relative to one another to position the distal tip portion 228 adjacent to a proximal end of the valve mounting portion 224, when the prosthetic valve 250 is mounted in the radially compressed state on the valve mounting portion 224 (as shown in FIG. 4) and during delivery of the prosthetic valve to the target implantation site. As such, the distal tip portion 228 can be configured to resist movement of the prosthetic valve 250 relative to the balloon 218 proximally, in the axial direction, relative to the balloon 218, when the distal tip portion 228 is arranged adjacent to a proximal side of the valve mounting portion 224.

An annular space can be defined between an outer surface of the inner shaft 208 and an inner surface of the intermediate shaft 206 and can be configured to receive fluid from a fluid source via the second port 240 of the adaptor 212. The annular space can be fluidly coupled to a fluid passageway formed between the outer surface of the distal end portion of the inner shaft 208 and an inner surface of the balloon 218. As such, fluid from the fluid source can flow to the fluid passageway from the annular space to inflate the balloon 218 and radially expand and deploy the prosthetic valve 250.

An inner lumen of the inner shaft can be configured to receive a guidewire therethrough, for navigating the distal end portion of the delivery apparatus 200 to the target implantation site.

The handle 202 can include a steering mechanism configured to adjust the curvature of the distal end portion of the delivery apparatus 200. In the illustrated example, for example, the handle 202 includes an adjustment member, such as the illustrated rotatable knob 260, which in turn is operatively coupled to the proximal end portion of a pull wire. The pull wire can extend distally from the handle 202 through the outer shaft 204 and has a distal end portion affixed to the outer shaft 204 at or near the distal end of the outer shaft 204. Rotating the knob 260 can increase or decrease the tension in the pull wire, thereby adjusting the curvature of the distal end portion of the delivery apparatus 200. Further details on steering or flex mechanisms for the delivery apparatus can be found in U.S. Patent No. 9,339,384.

The handle 202 can further include an adjustment mechanism 261 including an adjustment member, such as the illustrated rotatable knob 262, and an associated locking mechanism including another adjustment member, configured as a rotatable knob 278. The adjustment mechanism 261 is configured to adjust the axial position of the intermediate shaft 206 relative to the outer shaft 204 (e.g., for fine positioning at the implantation site). Further details on the delivery apparatus 200 can be found in PCT Application No. PCT/US2021/047056.

As introduced above, in some examples, an edge of an outer skirt of a prosthetic heart valve can be relatively rough or abrasive. For example, the edge can be a finished edge that is "molten" by melting fibers along the edge to reduce fraying of the fabric of the outer skit. In other examples, the skirt edge can have exposed fibers or loops. When the prosthetic heart valve is operating and the leaflets are in an open state, they can contact the edge (e.g., the outer or free edge) of the outer skirt. Such contact can, in some instances, cause unwanted abrasion or wear of the leaflets.

In some instances, in order to avoid the leaflets contacting the edge of the outer skirt during operation of the prosthetic heart valve, the edge of the outer skirt can be folded outward, away from the frame and the leaflets. The folded edge can then be secured to the frame with stitches that extend through the skirt's folded edge and around struts of the frame. In some examples, the stitches can pass through the folded skirt layers close to the edge of the skirt. Thus, pulling forces against the skirt from the stitches can cause fraying or unraveling of the skirt's edge.

Thus, the inventors herein have recognized that it is desirable to provide stitching lines along an edge portion (e.g., an outflow edge portion) of the outer skirt that allow the outer skirt to be attached to the struts of the frame such that the skirt edge is positioned away from the leaflets (such that it does not contact the leaflets during valve operation) and such that the skirt edge remains intact (e.g., doesn't fray or unravel).

FIGS. 6-13 illustrate exemplary methods for forming a stitching line along an edge portion of an outer skirt and attaching the edge portion of the outer skirt to struts of a frame of a prosthetic heart valve using the stitching line such that a free edge of the edge portion is positioned against outer surfaces of the struts and away from the leaflets of the prosthetic heart valve. The methods described below with reference to FIGS. 6-13 can be used for various combinations of frames and outer skirts. Thus, the outer skirts and frames shown in FIGS. 6-13 are exemplary and the disclosed methods can be applied to outer skirts and frames having different configurations, such as various balloon, self, and mechanically expandable frames and various skirts (which may have a straight, undulating, or alternatively shaped outflow edge portion or alternate edge portion). Further, though the methods below are described as being applied to an outflow edge portion of the outer skirt, in alternate examples, the stitching lines described herein can be used on another edge of the skirt (such as an inflow edge portion of the skirt which can be secured to an inflow end of a frame).

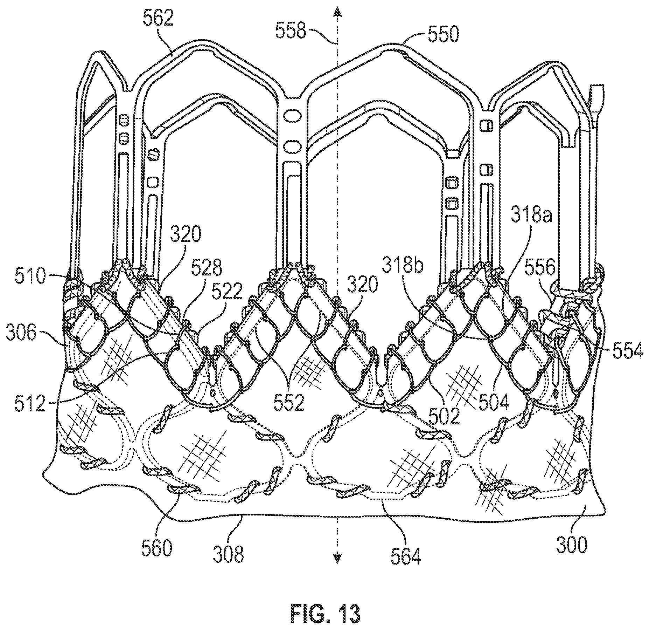

Turning first to FIG. 5, an exemplary outer skirt 300 for a prosthetic device, such as the prosthetic heart valve 100 of FIG. 1 (or the prosthetic heart valve frame 550 of FIG. 13), is shown in a flattened configuration. The outer skirt 300, as well as the other outer skirts disclosed herein, can be used in a balloon-expandable prosthetic valve (for example, valve 100 of FIGS. 1-3), a mechanically expandable prosthetic valve, and/or a self-expandable prosthetic valve. Additional details on balloon expandable prosthetic valves can be found in U.S. Patent No. 9,393,110 and PCT Application No. PCT/US2022/025687. Additional details on mechanically expandable prosthetic valves can be found in PCT Application PCT/US2021/052745. Additional details on a self-expanding prosthetic valve can be found in U.S. Patent No. 8,652,202.

The outer skirt 300 can be wrapped around and mounted to an outer surface of a frame of a prosthetic device (a radially outward facing surface relative to a central longitudinal axis of the prosthetic device), thereby transitioning to an annular configuration (e.g., as shown in FIG. 1 or FIG. 13).

As shown in FIG. 5, the outer skirt 300 can comprise opposing first and second edge portions 302, 304 (which can also be referred to as short edges or edge portions) which each extend between an outflow edge portion 306 and an inflow edge portion 308 of the outer skirt 300. In some examples, the first and second edge portions 302, 304 can be non-perpendicular to the inflow edge portion 308. For example, the first and second edge portions 302, 304 can extend at angles of about 45 degrees (or in a range of 40 to 50 degrees) relative to the inflow edge portion 308. Therefore, an overall general shape of the outer skirt 300 can be that of a rhomboid or parallelogram.

In some examples, the first and second edge portions 302, 304 can each comprise a plurality of apertures 310 extending therethrough. Thus, when the outer skirt 300 is converted into its annular configuration (e.g., when mounted around a prosthetic device, as shown in FIG. 13 for example), the first and second edge portions 302, 304 can overlap one another with their respective apertures 310 overlapping as well. A suture can then be used to form a plurality of stitches in and in-and-out pattern through the overlapping apertures 310, thereby securing the first and second edge portions 302, 304 together and forming the annular configuration of the outer skirt 300.

The outflow edge portion 306 (which is disposed closer to the outflow end of the prosthetic valve than the inflow edge portion 308 when arranged around the prosthetic valve, e.g., as shown in FIG. 13) can be formed with a plurality of projections 312 that define an undulating shape that generally follows the shape or contour of the struts of the frame to which they are secured (e.g., the struts 134 of the frame 102 in FIG. 2 or the struts 552 of the frame 550).

In alternate examples, the outflow edge portion 306 can have a different shape than shown in FIG. 5, such as relatively straight (e.g., parallel to the inflow edge portion 308, the same or similar to as illustrated in FIGS. 6-8B) or a different non-straight shape that does or does not follow the shape or contour of the struts of the frame to which they are secured.

In some examples, the outer skirt 300 can also be formed with slots 314 (or recesses) to facilitate attachment of the skirt to the frame. For example, each slot 314 can be arranged between two adjacent projections 312. In some examples, the slots 314 can be shaped and dimensioned such that the slots 314 fit against and/or can be wrapped or secured around the struts or support posts of the frame.

When the outer skirt 300 is arranged around an outer surface of the frame of the prosthetic device (e.g., as shown in FIG. 1 and FIG. 13) sutures can be used to secure the outflow edge portion 306 of the outer skirt 300 to the struts of the frame. In some examples, additional sutures can be used to secure the inflow edge portion 308 of the outer skirt 300 to the frame and/or leaflets of the prosthetic device (such as shown in FIG. 13, as described further below).

In some examples, the outer skirt 300 can comprise one or more rows of apertures 318 that can be configured to receive sutures of a stitching line therethrough (such as stitching line 402 shown in FIGS. 6-7B or stitching line 502 shown in FIGS. 8A-13). In alternative examples, the outer skirt 300 may not include pre-formed apertures 318 for the stitching line and instead the apertures or holes in a fabric of the outer skirt 300 can be formed by a needle during formation of the stitching line along the outflow edge portion 306.

The outer skirt 300 and other skirts or covering described herein can comprise various synthetic materials, including fabrics (e.g., polyethylene terephthalate fabric (PET fabric) or ultra high molecular weight polyethylene (UHMWPE) fabric)), polytetrafluoroethylene (PTFE), thermoplastic polyurethane (TPU), a hybrid material comprising one or more fabric or polymeric materials (e.g., PET coated in TPU), or natural tissue (e.g., pericardial tissue).

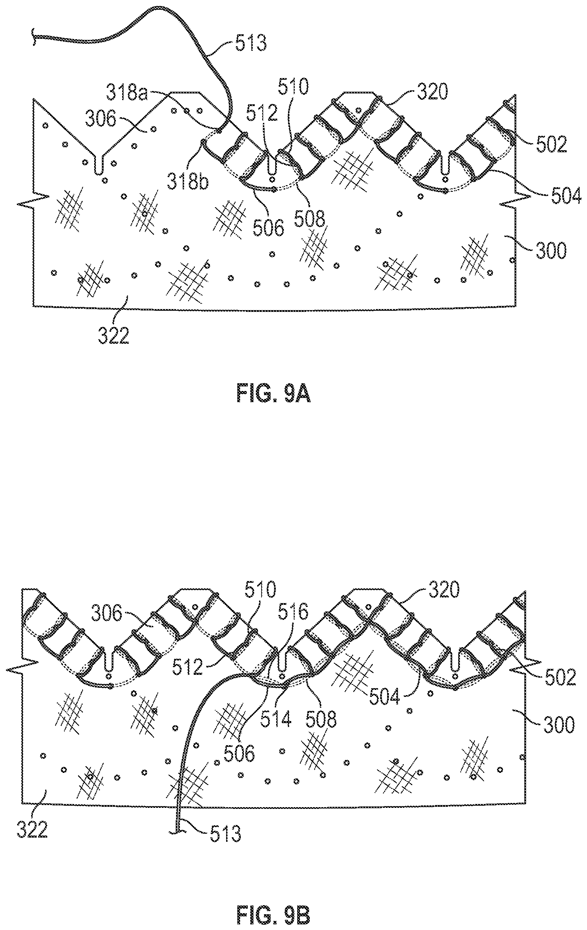

FIGS. 6-7B illustrate detail portions of the outer skirt 300 with a stitching line 402 arranged along the outflow edge portion 306. In FIGS. 6 and 7A, the outflow edge portion 306 is depicted as being relatively straight with a wavy outflow edge 320 (which can be referred to herein as a free edge or outer edge of the outer skirt 300). The outflow edge 320 is depicted as wavy for illustration purposes only and denotes that the edge 320 of the outflow edge portion 306 can be rough and/or molten (e.g., an edge formed by melting the fibers of the fabric skirt along the edge and/or an edge with exposed fibers or loops that may be abrasive, as described above). Additionally, though the outflow edge portion 306 is shown as being relatively straight in FIGS. 6 and 7A (for ease of illustration), in some examples, the outflow edge portion 306 can have an undulating edge (with projections 312, as shown in FIG. 5). In such examples, the stitching line 402 can follow the contour of the undulating outflow edge portion, similar to as shown in FIGS. 9A-10B and 12A-13, as described below.

The stitching line 402 comprises an in-and-out stitching line 404 spaced away from the outflow edge 320 of the outflow edge portion 306 (and comprising a plurality of in-and-out stitches 406, 408) and a plurality of loops 410 (which can also be referred to as bights or looped stitches) that loop over the outflow edge 320 and are spaced apart from one another in the circumferential direction (or in direction of the in-and-out stitches). For example, the loops 410 can be connected together within the stitching line 402 by the in-and-out stitches 406, 408.

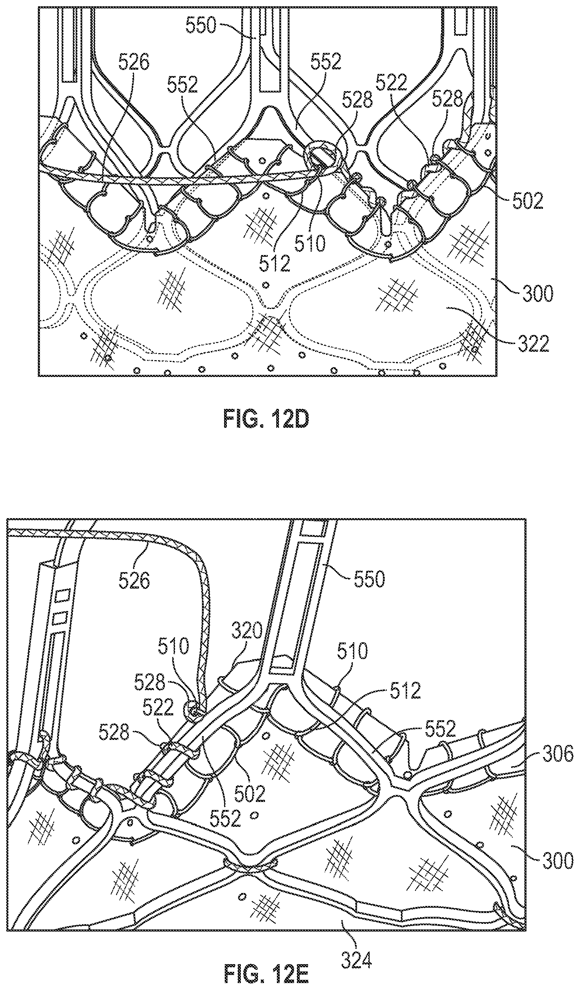

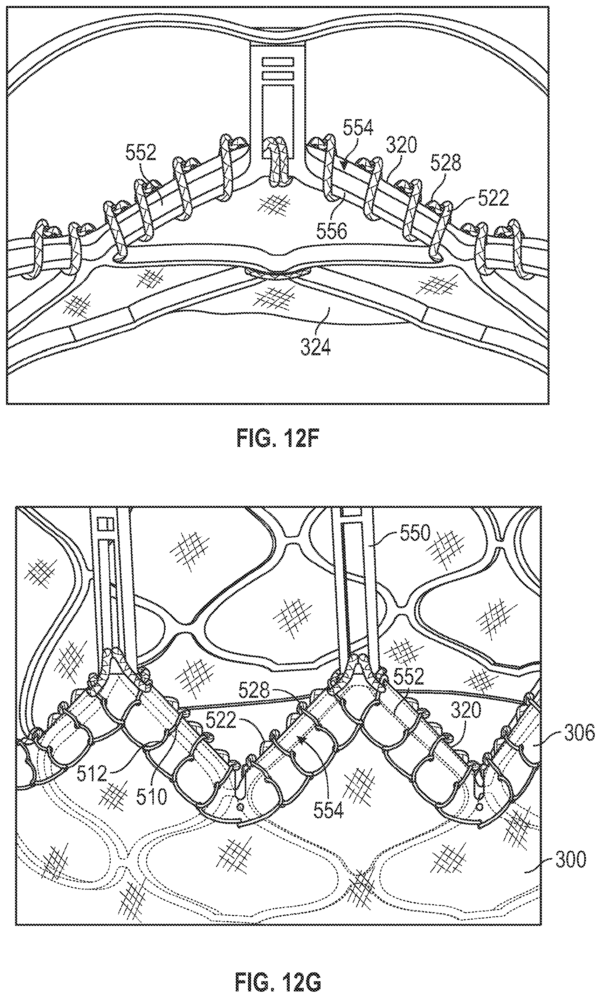

In some instances, the stitching line 402 can be formed by extending a suture 412, in a direction that is parallel to the outflow edge 320, over a first surface 322 (e.g., a radially outward facing surface which is facing out of the page in the views of FIGS. 5-7) of the outer skirt 300, from one aperture 318 to an adjacent aperture 318, thereby forming an in-and-out stitch 406 across the first surface 322. The suture 412 can then extend through the adjacent aperture 318, across a second surface 324 (e.g., a radially inward facing surface which is opposite the first surface 322 and can be seen in FIGS. 12E and 12F) of the outer skirt 300 toward the outflow edge 320, over the outflow edge 320, and back across the first surface 322 to the same aperture 318, thereby forming a loop 410. After passing back through the same aperture 318, the suture 412 can then extend across the second surface 324 (behind the fabric in the view of FIG. 6) to the next, adjacent aperture 318, thereby forming an in-and-out stitch 408 across the second surface 324 (the in-and-out stitches 408 are depicted in dashed lines in FIGS. 6 and 7A to denote their positioning across the second surface 324, or behind the outer skirt 300 in the views of FIGS. 6 and 7A). This process is then repeated to form the subsequent loops 410 and in-and-out stitches 406, 408, as shown in FIG. 6. For example, the method for forming the stitching line 402 described above is repeated in an alternating fashion across the first and second surfaces 322, 324 of the outer skirt 300 to form the plurality of in-and-out stitches 406, 408 and plurality of loops 410 of the stitching line 402. In some examples, this stitching pattern can be referred to as an overcast stitching pattern or stitch pattern. In FIG. 6, arrows are depicted showing an exemplary direction for stitching the stitching line 402, as described above.

In some instances, the in-and-out stitching line 404 can comprise a second pass of in-and-out stitches 414, 416 which comprise alternating stitches 414 extending across the first surface 322 and stitches 416 extending across the second surface 324 (shown in dashed lines in FIG. 7A). For example, the in-and-out stitches 414, 416 can alternate with the in-and-out stitches 406, 408 such that the in-and-out stitches 406, 408 and the in-and-out stitches 414, 416 extend across opposing surfaces of the outer skirt 300 (the first surface 322 and the second surface 324) between each corresponding pair of adjacent apertures 318.

The second pass of in-and-out stitches 414, 416 can be formed with the first suture 412 or a second suture. In this way, the in-and-out stitching line 404 can include an in-and-out stitch across both the first surface 322 and the second surface 324 between each pair of adjacent apertures 318.

As shown in FIG. 6, the in-and-out stitching line 404 is spaced apart from the outflow edge 320 by a distance 418. The distance 418 can be specified such that the in-and-out stitching line 404 is spaced far enough away from the outflow edge 320 to prevent fraying or unraveling of the outflow edge 320. In some examples, the distance 418 can be in a range of about 0.5 - 2 mm, 1.0 - 2.0 mm, 1.5 - 2.0 mm, or 1.4 - 2.1 mm. Thus, the loops 410 can have a length that is similar to and/or results in the distance 418.

As shown in FIGS. 7A and 7B, additional stitches 420 (e.g., whip stitches) can extend through the loops 410 of the stitching line 402 and around a strut 422 (or struts) of the prosthetic valve frame (where strut 422 can be replaced by a strut 134 or struts 134 of frame 102 or strut 552 of the frame 550), thereby attaching the outflow edge portion 306 of the outer skirt 300 to the strut(s) 422 via the stitching lines 402. As a result, pulling forces on the outer skirt 300 from the stitches 420 can be transferred away from the outflow edge 320, and to the in-and-out stitching line 404 by the loops 410. Since the in-and-out stitching line 404, which is spaced away from the outflow edge 320 by the distance 418, receives the pulling forces, the integrity of outflow edge 320 can be preserved, thereby increasing a longevity of the outer skirt 300.

Further, as shown in FIG. 7B, such an attachment method can result in the outflow edge 320 being position behind or on a radially outward facing surface 424 of the struts 422 (e.g., a surface that faces away from the leaflets which are arranged on an interior of the frame, closer to the opposite, radially inward facing surface 426 of the strut 422), thereby concealing the outflow edge 320 away from the leaflets of the prosthetic valve. It should be noted that the stitches 420 are shown in an untightened stage in FIG. 7A for illustration purposes only. Upon tightening of the stitches 420, the outflow edge 320 is positioned against the radially outward facing surface 424 of the strut 422, as shown in FIG. 7B.

FIGS. 8A-9B show another example of a stitching line 502 disposed in and along the outflow edge portion 306 of the outer skirt 300. The stitching line 502 can be similar and implemented similarly to the stitching line 402, as described above, except the loops 410 are replaced by double loops 510, 512 (for example, two loops in a figure-eight pattern, as explained in further detail below). In some instances, the outer skirt 300 can include an additional row of pre-formed apertures 318 to receive the double loops 510, 512 (such as a first aperture 318a and second aperture 318b, as shown in FIGS. 8A and 8B). In some instances, the additional row of apertures may not be pre-formed, and instead can be formed by stitching with a needle through a fabric of the outer skirt 300. For example, as shown in FIGS. 9A and 9B the second apertures 318b may not be pre-formed and may instead be formed as the stitching line 502 is formed (for example, as a needle holding the suture extends through the fabric of the outer skirt 300 at the locations shown in FIGS. 9A and 9B).

For ease of illustration, the outflow edge 320 and outflow edge portion 306 are shown as being relatively straight in FIGS. 8A and 8B. However, FIGS. 9A and 9B depict the outer skirt 300 with an undulating outflow edge portion 306 and outflow edge 320 (as described above with reference to FIG. 5). The stitching line 502 can be applied to an outer skirt having a straight, undulating, or differently shaped edge portion using the same methods described below with reference to FIGS. 8A-13.

Similar to the stitching line 402, the stitching line 502 comprises an in-and-out stitching line 504 spaced away from the outflow edge 320 of the outflow edge portion 306 and a plurality of pairs of loops that are spaced apart from one another in a direction of the in-and-out stitches (for example, a circumferential direction and/or a direction that is parallel to the outflow edge 320). The in-and-out stitching line 504 comprises a plurality of in-and-out stitches 506, 508. Each pair of loops includes a first loop 510 and a second loop 512, the first loop 510 circling around the outflow edge 320 of the outflow edge portion 306 and extending between the outflow edge 320 and the second loop 512, and the second loop 512 extending between the first loop 510 and an intersection of two adjacent in-and-out stitches 506, 508 of the plurality of in-and-out stitches 506, 508 of the in-and-out stitching line 504. The second loops 512 are connected together within the stitching line 504 by the in-and-out stitches 506, 508.

In some instances, the stitching line 502 can be formed by extending a first suture 513, in a direction that is parallel to the outflow edge 320, over the first surface 322 of the outer skirt 300, from one second aperture 318b to an adjacent second aperture 318b, thereby forming an in-and-out stitch 506 across the first surface 322 (FIGS. 8A and 9A). The suture 513 can then extend through the adjacent second aperture 318b, across the second surface 324 of the outer skirt 300, through a first aperture 318a (which is closer to the outflow edge 320 that the second aperture 318b), across the first surface 322 of the outer skirt 300 toward the outflow edge 320, over the outflow edge 320, back across the second surface 324 to the same first aperture 318a, through the first aperture 318a, and back across the first surface 322 to the same second aperture 318b. As a result, a first loop 510 and second loop 512 are formed between the outflow edge 320 and the in-and-out stitching line 504. As shown in FIGS. 8A-9B, the first loop 510 and the second loop 512 are connected to one another. The first loop 510 and second loop 512 can be stitched in and form a figure-eight pattern across the outflow edge portion 306, as described above. The crossing or figure-eight stitching pattern for the first loops 510 and second loops 512 can help to prevent lateral (or circumferential when the outer skirt is arranged around the annular frame) displacement of the first loop 510 and/or the second loop 512 (which can better maintain the whip stitches looping through the first loops 510 in place, as described further below).

After passing back through the same second aperture 318b, the suture 513 can then extend across the second surface 324 (behind the fabric in the view of FIGS. 8A-9B) to the next, adjacent second aperture 318b, thereby forming an in-and-out stitch 508 across the second surface 324 (the in-and-out stitches 508 are depicted in dashed lines in FIGS. 8A-9B to denote their positioning across the second surface 324, or behind the outer skirt 300 in the views of FIGS. 8A-9B). This process is then repeated (but passing over the opposite surfaces of the skirt 300 than as described above for each stitch, in an alternating fashion) to form the subsequent first and second loops 510, 512 and in-and-out stitches 506, 508, as shown in FIGS. 8A and 9A. In some instances, this stitching pattern can be referred to as an overcast stitching or stitch pattern.

In some instances, the in-and-out stitching line 504 can comprise a second pass of in-and-out stitches 514, 516 which comprise alternating stitches 514 extending across the first surface 322 and stitches 516 extending across the second surface 324 (shown in dashed lines in FIGS. 8A and 9A). In some instances, the second pass of in-and-out stitches 514, 516 can be formed with the first suture 513. In some instances, the second pass of in-and-out stitches 514, 516 can be formed with a different, second suture. In this way, the in-and-out stitching line 504 can include an in-and-out stitch across both the first surface 322 and the second surface 324 between each pair of adjacent second apertures 318b.

As shown in FIGS. 8A and 8B, the in-and-out stitching line 504 is spaced apart from the outflow edge 320 by a distance 518. The distance 518 can extend in a direction that is perpendicular to the outflow edge 320. The distance 518 can be specified such that the in-and-out stitching line 504 is spaced far enough away from the outflow edge 320 to prevent fraying or unraveling of the outflow edge 320. The first loops 510 have a length 520 (which may be the same or similar to a distance between the outflow edge 320 and the first apertures 318a). In some instances, the length 520 is smaller than a distance between the first aperture 318a and the second aperture 318b (e.g., a length of the second loop 512 or a difference between the distance 518 and the length 520).

For example, in some instances, the distance 518 can be about 2 mm (for example, ± 0.1 mm) and the length 520 of the first loops 510 can be about 0.5mm (and thus the second loops 512 can be about 1.5 mm in length). In some instances, the distance 518 can be in a range of 1.5 - 2.0 mm and the length 520 of the first loops 510 can be about 0.3 mm (and thus the second loops 512 can be about 1.2 - 1.7 mm in length). In some instances, the distance 518 can be in a range of 1.4 - 2.1 mm and the length 520 of the first loops 510 can be in a range of 0.2 - 0.6 mm (and thus the second loops 512 can be about 0.8 - 1.9 mm in length.

FIGS. 10A-10B show an exterior view (FIG. 10A) and interior view (FIG. 10B) of a frame 550 for a prosthetic heart valve, with the outer skirt 300 with the stitching line 502 arranged around an outer surface of the frame 550. The frame 550 can be similar to the frame 102 of FIG. 2, as described above. In some examples, the frame 102 of FIG. 2 can be used in lieu of the frame 550 in FIGS. 10A-13. Further, as described above, the outer skirt 300 with the stitching line 502 can be attached to various other frames using the methods described herein (for example, frames having different numbers of rows of struts, differently shaped cells or struts, or the like).

As shown in FIG. 10B, additional stitches 522 (which can be referred to as whip stitches) extend through the first loops 510 of the stitching line 502 and around struts 552 of the frame 550 in order to attach the outflow edge portion 306 of the outer skirt 300 to the struts 552 via the stitching line 502. In some instances, the struts 552 can be angled struts of a second row of angled struts of the frame 550 that are disposed adjacent to a first row of angled struts that define an outflow end of the struts (for example, the same or similar to struts 134 of the frame 102, and as shown in FIG. 13 showing the full frame 550).

For example, as shown in FIG. 10A, a needle 524 (such as a blunt needle without a sharp tip) can be used to form the whip stitches 522 with a suture 526, by passing the needle 524 and the suture 526 through a first loop 510 and then around one of the struts 552, without penetrating a material of the outer skirt 300 (this process is then repeated around the frame 550 and outer skirt 300). Said another way, the whip stitches 522 pass through the first loops 510 only and not through another portion of the outer skirt 300. Further, the in-and-out stitching line 504 is offset (or spaced away) from the whip stitches 522, at least by the length of the second loops 512 (which can be the same or similar to the difference between the distance 518 and the length 520).

As a result, pulling forces on the outer skirt 300 from the whip stitches 522 can be transferred away from the outflow edge 320, and to the in-and-out stitching line 504 by the first loops 510 and the second loops 512. Since the in-and-out stitching line 504, which is spaced away from the outflow edge 320 by the distance 518, receives the pulling forces, the integrity of outflow edge 320 can be preserved, thereby increasing a longevity of the outer skirt 300.

By including the first loops 510, in addition to the second loops 512, lateral motion of the second loops 512 relative to the outer skirt 300 can be prevented. Additionally, the pair of loops configuration of the stitching line 502 can prevent motion of the whip stitches 522 along the first loops 510 in the vertical or axial direction (which can be a direction of a central longitudinal axis 558 of the frame 550, as depicted in FIG. 13).

As shown in FIG. 10B, by passing through the first loops 510 and around the struts 552, the whip stitches 522 can prevent the outer skirt 300 from sliding over the whip stitches 522 and maintain the outflow edge 320 positioned behind (in the view of FIG. 10B) or on a radially outward facing surface 554 of the struts 552 (e.g., a surface that faces away from the leaflets which are arranged on an interior of the frame), thereby concealing the outflow edge 320 away from the leaflets of the prosthetic valve.

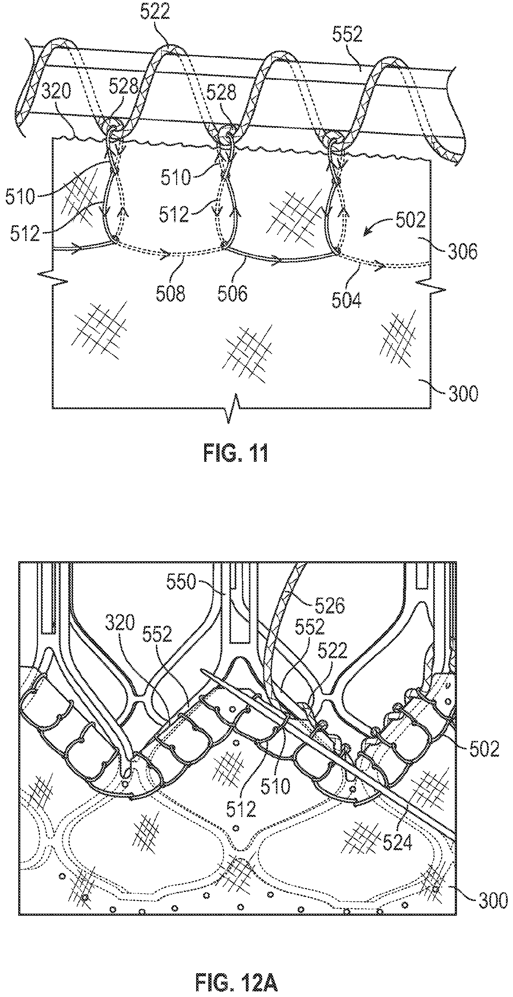

In some examples, one or more of the whip stitches 522 can be double-looped around the corresponding first loop 510. For example, at least on whip stitch 522 along a same strut 552 can be double-looped around the corresponding first loop 510. In some instances, more than one or all of the whip stitches 522 along the same strut 552 can be double-looped around their corresponding first loops 510. FIG. 11 is a schematic showing a plurality of whip stitches 522 around the strut 552 formed with a double-loop 528 around the corresponding first loops 510. It should be noted that the whip stitches 420 shown in FIG. 11 can also be double-looped around the loops 410, in the same way as described below.

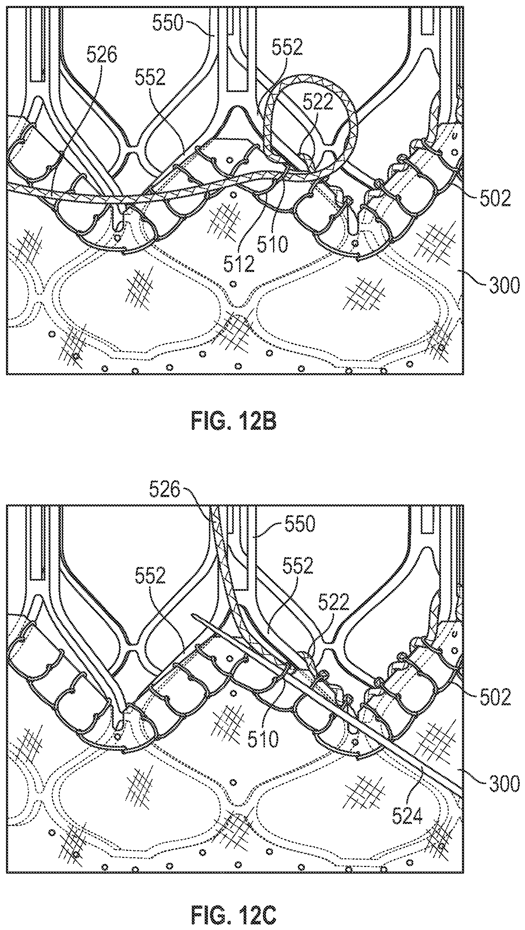

FIGS. 12A-12G show an exemplary process for forming the whip stitches 522 through the first loops 510 and around the frame struts 552 with the double-loop 528. For a first whip stitch 522, the suture 526 used to form the whip stitches 522 is first passed (using the needle 524, for example) from around the strut 552 (FIG. 12A), through the first loop 510 (FIG. 12B), and is then wrapped over the edge of the first loop 510 and back through the same first loop 510 a second time (as shown in FIG. 12C where the needle 524 going through the first loop 510 the second time and FIG. 12D which shows the second loop of the double-loop 528 being formed in the first loop 510). Finally, the suture extends back toward and around the strut 552, and this process can be repeated on the next whip stitch 522 (or a select number of the following whip stitches 522).

FIG. 12E shows an interior view of the frame 550, with the whip stitches 522 and their double-loops 528 being formed along the outflow edge portion 306 of the outer skirt 300. FIG. 12F shows another interior view of a smaller portion of the frame 550, with double-loops 528 at each whip stitch 522. As shown in FIG. 12F and the exterior view of FIG. 12G, the double-loops 528 of the whip stitches 522 tighten around the first loops 510 and pull the outflow edge 320 of the outer skirt 300 against radially outward facing surfaces 554 of the struts 552 (which are opposite radially inward facing surfaces 556 of the struts 552). As a result, the outflow edge 320 of the outer skirt 300 can be positioned away from the leaflets of the prosthetic heart valve (which are disposed on an interior of the frame and face the radially inward facing surfaces 556 of the struts 552).

FIG. 13 is a perspective view of the full frame 550 with the outer skirt 300 attached to the outer surface of the frame 550 using the stitching line 502 and the whip stitches 522 with the double-loop 528 (as described above with reference to FIGS. 11-12G). As shown in FIG. 13, the outflow edge portion 306 of the outer skirt 300 is secured to the angled struts 552 and the outflow edge 320 remains on an exterior of the frame, on the radially outward facing surfaces 554 of the struts 552. As such, when leaflets are arranged within an interior of the frame 550 (the same or similar to as shown in FIG. 1), the outflow edge 320 is separated from the leaflets by the struts 552, thereby preventing wear on the leaflets from the outflow edge 320 during operation of the prosthetic heart valve.

As shown in FIG. 13, the struts 552 can define a first circumferentially extending row of struts of the frame 550 and the frame 550 can further comprise a second circumferentially extending row of outflow struts 562 that define an outflow end of the frame 550 and are disposed adjacent to the row of struts 552. The frame 550 can further include a third circumferentially extending row of inflow struts 564 that define an inflow end of the frame 550. The frame 550 can include one or more additional circumferentially extending rows of struts (such as an additional row of struts disposed between the inflow struts 564 and struts 552, as shown in FIG. 13). As shown in FIG. 13, the inflow edge portion 308 of the outer skirt 300 is secured to the inflow struts 564 via a plurality of whip stitches 560 that extend through the outer skirt and around the inflow struts 564 (without an additional stitching lines). In alternate examples, the stitching line 502 or 402 can be applied to the inflow edge portion 308 and used to secure the inflow edge portion 308 to the inflow struts 564, similar to as described above with reference to FIGS. 8A-12G or FIGS. 6-7B.

In some instances, not all whip stitches 522 that connect the stitching line 502 to the frame 550 include the double-loop 528. For example, one or only a portion of whip stitches along a same strut 552 may include the double-loop 528, while the remaining whip stitches 522 along the same strut 552 include only a single loop. In some examples, only a portion of the struts 552 of the row of struts 552 may include whip stitches 522 with the double-loop 528. The number of whip stitches along the same strut 552 that include the double-loop 528 can be specified such that the outflow edge portion 306 of the outer skirt 300 is tightly attached to the frame 550 in a manner that conceals the outflow edge 320 on the outer surface of the frame 550 (with the struts 552 separating the outflow edge 320 and the leaflets from one another).

In some examples, the outer skirt 300 can be attached to the frame 550 (or another, similar frame, such as the frame 102 of FIG. 2) with the stitching line 502, without the double-loops 528 (as shown in FIGS. 8A-10B) or with the stitching line 402 (for example the strut 422 shown in FIGS. 7A and 7B can instead be one of the struts 552 of the frame 550).

The frame 550 is exemplary, and in some examples, the frame 102 of FIG. 2 can be used in lieu of the frame 550 with the outer skirt 300 being attached to the struts 134 in the same way as shown in FIGS. 11-13, FIGS. 8A-10B, or FIGS. 6-7B.

The methods described herein for attaching an edge portion of an outer skirt to struts of a frame of a prosthetic heart valve can be applied to various frames for prosthetic heart valve or alternative implantable medical devices (such as stents). Additionally, the method described herein can be applied to a variety of skirts and skirt edge portions, such as edges with various shapes (straight, undulating, or an alternative shape) and edges configured to be attached to various points of a frame (for example, the outflow end, the inflow end, or a portion of the frame disposed between the inflow and outflow end).

The methods described herein for attaching an edge portion of an outer skirt to struts of a frame using a stitching line comprising one or more loops that offset in-and-out stitches of the stitching line from a free edge of the edge portion result in the free edge being positioned against surfaces (for example, radially outward facing surfaces) of the struts that are opposite surfaces of the struts that face leaflets or other soft components arranged on an interior of the frame, thereby preventing the free edge of the skirt from contacting the leaflets. Additionally, the stitching lines described herein allow the loads or pull forces from the whip stitches (which extend around the struts and through the loops of the stitching lines) to be transferred away from the free edge of the skirt, thereby reducing a risk of fraying of the free edge and increasing a durability and longevity of the outer skirt. Further still, by using the stitching lines and methods described herein, the edge portion of the outer skirt can be attached to the frame without folding, thereby allowing the stitching processes on the skirt to be performed in a flattened configuration (off the frame). As a result, since the stitches are all in the same plane, the stitching of the outer skirt (to form the stitching line) can be automated, thereby reducing assembly times and increasing the efficiency and accuracy of assembling the outer skirt and the prosthetic device.

In some examples, a stitching line comprising a plurality of in-and-out stitches and a plurality of loops that are spaced apart from one another in a direction of the in-and-out stitches can be applied to different or additional components of a prosthetic valve, such as leaflets of a valvular structure. Such stitching lines can be disposed along cusp edge portions of the leaflets with the loops looping around cusp edges of the leaflets and spacing the in-and-out stitches away from the cusp edges. In some examples, the cusp edge portions of the leaflets can then be attached to an additional component, such as a fabric skirt, sutures extending around struts of the frame, or directly to the struts of the frame, using the stitching line and without piercing the tissue of the leaflets. As a result, the cusp edge portions of the leaflets can be secured to the frame of the prosthetic valve in a manner than tightly tucks the cusp edges to the additional component and reduces blood flow disturbances through the prosthetic valve.

FIGS. 14-16 show an example of a stitching line 612 formed along a cusp edge portion 602 of an exemplary leaflet 600 for a prosthetic valve. In some examples, the leaflet 600 can be one of the leaflets 112 of FIG. 1. The leaflet 600 comprises an outflow edge 604, two opposing commissure tabs 606 disposed on opposite sides of the leaflet 600, and the cusp edge portion 602 extending between the opposing commissure tabs 606. The cusp edge portion 602 comprises an outermost cusp edge 608 (or free cusp edge). The cusp edge 608 of the leaflet 600 can have an undulating, curved scalloped shape, and can form an inflow edge or inflow end of the leaflet 600. It should be noted that the leaflet 600 is exemplary and the stitching line 612 can be applied to the cusp edge portion of various leaflets, such as leaflets having differently shaped cusp edge portions, commissure tabs, outflow edges, and/or the like.

The stitching line 612 comprises an in-and-out stitching line 614 spaced away from the cusp edge 608 of the cusp edge portion 602, the in-and-out stitching line comprising a plurality of in-and-out stitches 616, 618. The in-and-out stitches 616, 618 can extend in a direction that is tangent or parallel to the cusp edge 608. The stitching line 612 further comprises a plurality of loops 620 (which can also be referred to as bights or looped stitches) that loop over the cusp edge 608 and are spaced apart from one another in a direction of the in-and-out stitches 616, 618. For example, the loops 620 can be connected together within the stitching line 612 by the in-and-out stitches 616, 618.

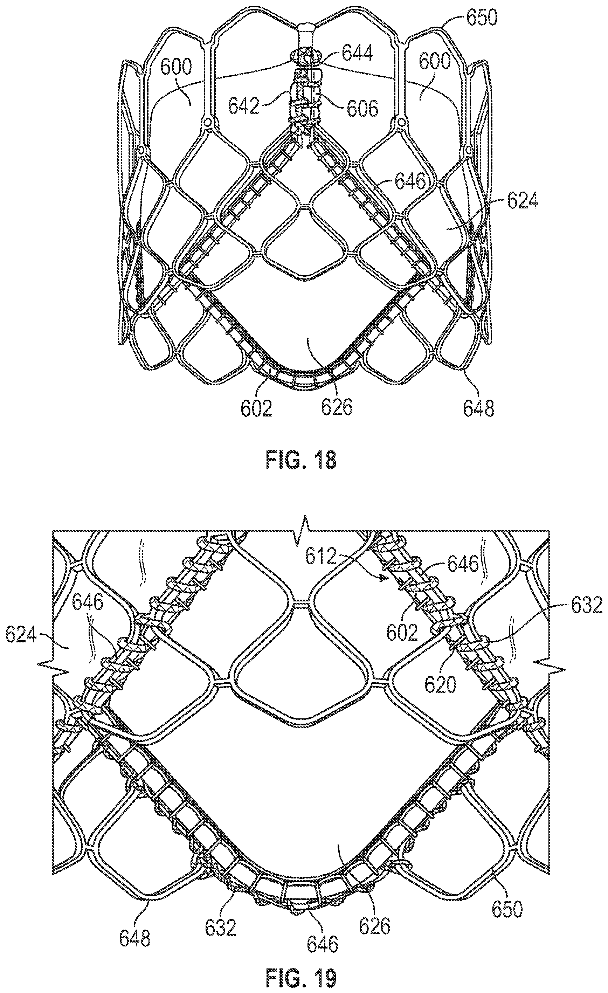

In some instances, the stitching line 612 can be formed by extending a suture 622, in a direction of the cusp edge portion 602, over a first side 624 (or surface) (e.g., a rough or smooth side of the leaflet 600) of the leaflet 600, and through the cusp edge portion 602 to an opposite, second side 626 of the leaflet 600 (as shown in FIGS. 18-21), thereby forming an in-and-out stitch 616 across the first side 624. The suture 622 can then extend across the second side 626 (the side "behind" the first side 624 in the view of FIGS. 14-16) of the leaflet 600 toward the cusp edge 608, over the cusp edge 608, and back across the first side 624 to the same in-and-out stitch 616, thereby forming a loop 620. After passing back through the same aperture created by the in-and-out stitch 616, the suture 622 can then extend across the second side 626 and through the cups edge portion 602 back to the first side 624, thereby forming an in-and-out stitch 618 across the second side 626 (the in-and-out stitches 618 are depicted in dashed lines in FIGS. 14 and 15 to denote their positioning across the second side 626, or behind the leaflet 600 in the views of FIGS. 14 and 15). This process is then repeated to form the subsequent loops 620 and in-and-out stitches 616, 618, as shown in FIG. 14.

For example, the method for forming the stitching line 612 described above is repeated in an alternating fashion across the first and second sides 624, 626 of the leaflet 600 along the cusp edge portion 602 (e.g., from a first end 610a of the cusp edge portion 602 disposed adjacent to one commissure tab 606 to a second end 610b of the cusp edge portion 602 disposed adjacent to the opposite commissure tab 606 of the leaflet 600, as depicted in FIG. 16) to form the plurality of in-and-out stitches 616, 618 and plurality of loops 620 of the stitching line 612. In some examples, this stitching pattern can be referred to as an overcast stitching pattern or stitch pattern. In FIGS. 14 and 15, arrows are depicted showing an exemplary direction for stitching the stitching line 612, as described above.

In some examples, the cusp edge portion 602 can comprise a plurality of pre-formed apertures through which the in-and-out stitches 616, 618 extend, between the first side 624 and second side 626 of the leaflet 600.

In some instances, the in-and-out stitching line 614 can comprise a second pass of in-and-out stitches which comprise alternating stitches 628 extending across the first side 624 and the second side 626 of the leaflet (as shown in FIG. 16). For example, the in-and-out stitches 628 can alternate with the in-and-out stitches 616, 618 such that the in-and-out stitches 616, 618 and the in-and-out stitches 628 extend across opposing surfaces of the leaflet 600 (the first side 624 and the second side 626) between each pair of loops 620.

The second pass of in-and-out stitches 628 can be formed with the first suture 622 or a second suture (as shown in FIG. 16). In this way, the in-and-out stitching line 614 can include an in-and-out stitch across both the first side 624 and the second side 626 between each pair of adjacent loops 620.

As shown in the detail view 625 of FIG. 15, the in-and-out stitching line 614 is spaced apart from the cusp edge 608 by a distance 630. In some instances, the distance 630 can be the same or similar to the distance 418 described above. For example, in some examples, the distance 630 is in a range of 0.5 - 2.0 mm.

In some instances, the distance 630 can be specified such that the length of the loops 620 is sufficient to receive a stitch therethrough and secure the cusp edge portion 602 to another component in a manner that tucks the cusp edge 608 to the component and reduces flow disturbances across the surface of the leaflet 600.

In some examples, as shown in FIG. 16, one or more main sutures (referred to herein as a thicker suture or first suture) 636 can be placed along the cusp edge portion 602 of the leaflet. In some instances, the main suture(s) 636 is thicker than the suture 622 forming the stitching line 612. The in-and-out stitches 616, 618, and/or 628 of the in-and-out stitching line 614 can extend through the main suture(s) 636, thereby securing the main suture(s) 636 to the cusp edge portion 602.

In some examples, a single main suture 636 can extend along the first side 624 of the leaflet 600 and then wrap around the first end 610a and extend along the second side 626 of the leaflet 600. In this way, the main suture 636 can extend along the cusp edge portion 602 on both sides of the leaflet 600.

In some examples, two main sutures 636 can extend along the cusp edge portion 602, one along the first side 624 and another along the second side 626.

Suture tails 638 of the main suture 636 (or two first sutures 636) are depicted in FIG. 16, and in some examples can be used to form a wedge member 640 (FIG. 17), as described further below. Further details on the main sutures 636 and wedge members 640 formed from suture tails 638, and additional examples of such configurations, are described further below with reference to FIGS. 22 and 23 and in U.S. Provisional Application No. 63/343,359.

As shown in FIGS. 14 and 15, additional stitches 632 (e.g., whip stitches) can extend through the loops 620 of the stitching line 612 and around or through a target component 634 in a manner that tightly couples the cusp edge 608 of the leaflet 600 to the target component 634 when the stitches 632 are pulled tight (they are depicted more loosely in FIGS. 14 and 15 for the purpose of illustration only).

In some examples, the target component 634 can be a fabric component, such as a skirt (e.g., inner skirt) or strip of cloth or attachment member configured to be secured on an inside of the frame of the prosthetic valve. For example, after stitching the cusp edge portions 602 of the leaflets 600 to the fabric component, the fabric component can then be attached to struts of the frame, on an inside of the frame, thereby securing the leaflets 600 to the frame. In some examples, the inner skirt is attached to a portion of angled struts that extend from a first commissure support portion (e.g., commissure support portion 644 shown in FIG. 18 or axially extending window struts 138 shown in FIGS. 1-3) of the frame to the inflow end of the frame and from the inflow end of the frame to a second commissure support portion (e.g., commissure support portion 644 shown in FIG. 18 or axially extending window struts 138 shown in FIGS. 1-3) of the frame, where the first and second commissure support portions are disposed adjacent to an outflow end of the frame.

In some instances, the skirt or fabric attachment member can comprise a plurality of pre-formed apertures that are configured to receive the whip stitches 632 therethrough. In some instances, the apertures in the skirt or fabric attachment member may not be pre-formed and instead can be formed as a needle threads the whip stitches 632 through the skirt or fabric attachment member.

In some examples, the target component 634 can be struts of the frame and/or a suture directly wrapped around struts of the frame (e.g., the struts following the scallop line of the leaflet and extending between adjacent commissure support portions of the frame and an inflow end of the frame).

An exemplary method for attaching leaflets 600 directly to struts of a frame (or directly to the struts via sutures wrapped around the struts of the frame) using the stitching line 612 is described below with reference to FIGS. 17-21. Though the leaflets 600 are depicted in FIGS. 18-21 as being attached to a frame 650, the leaflets 600 can be attached in a similar manner to struts of various other frames, such as frame 102, frame 550, or a differently configured prosthetic valve frame. In some examples, the frame 650 can be the same or similar to the frame 102, except it includes an additional circumferentially extending row of angled struts, thereby forming an additional row of cells of the frame 650.

As shown in FIG. 17, a plurality of leaflets 600 (e.g., three) can be assembled together in a flattened configuration (off the frame). In some examples, the stitching line 612 can be formed along the cusp edge portion 602 of each leaflet 600 (for example, with the in-and-out stitches 616, 618, 628 extending through the leaflet 600 and the main suture 636 extending along both sides of the cusp edge portion 602), as described above with reference to FIGS. 16.

In some instances, the stitching lines 612 of the plurality of leaflets 600 can be connected to one another.

In some instances, the suture tails 638 of the main sutures 636 can be woven or threaded together at adjacent commissure tabs 606 of adjacent leaflets 600 to form wedge members 640 (FIG. 17).

After forming the leaflet or valvular assembly show in FIG. 17, the leaflets 600 can then be mounted within the frame 650 and attached directly to the struts of the frame 650. In particular, FIG. 18 shows the leaflets 600 mounted within an interior of the frame 650 and commissures 642 secured to commissure support portions 644 (e.g., axially extending window struts, such as the axially extending window struts 138 of frame 102) of the frame 650. Each commissure 642 can be formed by securing adjacent commissure tabs 606 together around a wedge member 640 and to the commissure support portions 644 of the frame 650.

The stitching lines 612 can then be attached to angled struts 646 that follow the cusp edge portions 602 of the leaflets 600 and extend between one commissure support portion 644 to an inflow end 648 of the frame 650, and from the inflow end 648 to an adjacent commissure support portion 644. For example, the stitching lines 612 can be attached to the angled struts 646 by extending whip stitches 632 through the loops 620 of the stitching lines 612 and around the struts 646, as depicted in FIGS. 19-21.

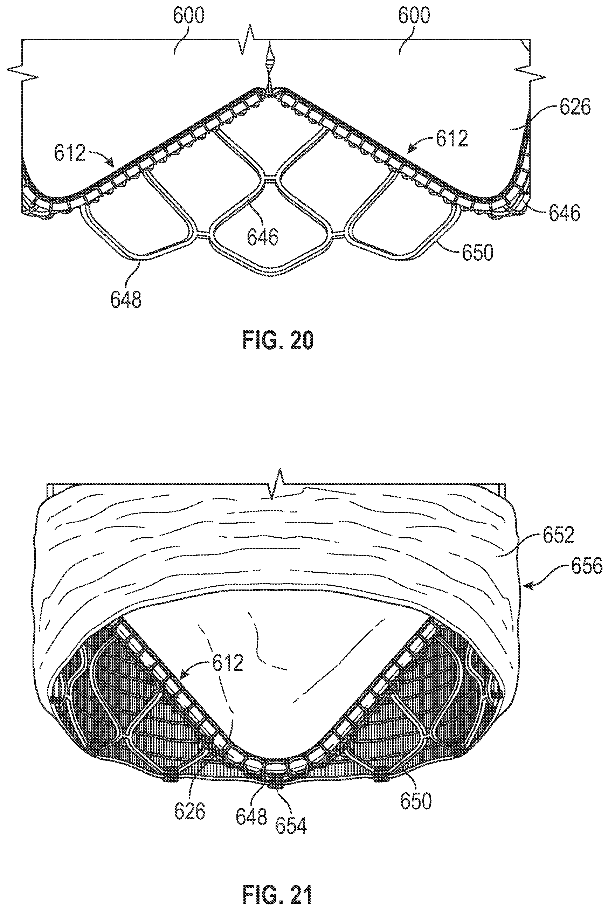

In some examples, after securing the leaflets 600 to the struts 646 of the frame 650 on an interior of the frame 650, an outer skirt 652 (or sealing member) can be attached to the frame 650 around an outer surface of the frame 650, as shown in FIG. 21.

In some instances, stitches 654 can be used to secure an inflow edge of the outer skirt 652 to angled struts 646 at the inflow end 648 of the frame 650 (e.g., to inflow apex regions of the frame 650).

In some cases, interlocking of the whip stitches 632 (e.g., passing the whip stitches 632 through the suture forming the whip stitches 632) may be utilized to ensure stability of the loops 620 (e.g., to keep the loops 620 from traveling or moving along the cusp edge 608).

In some instances, a double-loop can be formed with the whip stitches 632 around the loops 620 (similar to as shown in FIG. 11 for double-loops 528), thereby preventing the loops 620 from sliding along the whip stitches 632.

While the prosthetic valve 656 in FIG. 21 is shown with only an outer skirt 652, it is to be understood that in some examples, the leaflets 600 can be sutured along their cusp edge portions 602 to an inner skirt or fabric strip using the stitching lines 612 and whip stitches, in a similar manner as described above (instead of directly to the struts of the frame 650).

Advantageously, the above-described stitching configuration utilizing the stitching lines 612 and whip stitches 632 tightly tucks the cusp edges 608 of the leaflets 600 to the inner surfaces of the frame struts (or alternatively to an inner skirt or fabric) to which they are attached, without forming distally extending free portions between a suture line and the cusp edges 608 (e.g., without formed a stepped interface). As a result, a relatively continuous geometry along the inflow aspect of the leaflets if formed, thereby reducing the likelihood of blood flow disturbances along these inflow regions.

In some examples, a connecting suture line formed along a cusp edge portion of a leaflet can be used to secure the leaflet to a frame of a prosthetic heart valve and/or additional components of the prosthetic heart valve, as described below with reference to FIGS. 22-26.

Turning first to FIGS. 22 and 23, leaflets 702 are shown secured together into an exemplary leaflet assembly 700 (or valvular assembly) with a connecting suture line 704 disposed along a cusp edge portion of each of the leaflets 702. As described further below, the connecting suture line 704 is configured to enable securing of the leaflet assembly 700 directly to a frame of a prosthetic heart valve (e.g., the frame 102 of FIGS. 1-3, as shown in FIGS. 24-26), without having to extend or thread sutures through the material of the leaflet 702 during assembly to the frame.

As used herein, a "connecting suture line" can be defined as a line of multiple sutures that extends along the cusp edge portion on at least one side of at least one leaflet and that comprises at least one first suture extending along the cusp edge portion on the at least one side of the at least one leaflet and a plurality of in-and-out stitches (e.g., formed with a second suture) that extend through the at least one first suture and the cusp edge portion of the leaflet. The in-and-out stitches can secure the first suture to the leaflet. The connecting suture line can be formed on the leaflet while the leaflet is off the frame. Further, the connecting suture line can be formed (e.g., stitched) manually or automatically.

Returning to FIGS. 22 and 23, each leaflet 702 can comprise opposing commissure tabs 706 disposed on opposite sides of the leaflet 702 and a cusp edge portion 708 extending between the opposing commissure tabs 706 (similar to the leaflet 600 described above). FIG. 23 shows a single leaflet 702 while FIG. 22 shows a flat view of the leaflet assembly 700 comprising three leaflets 702 secured together along their cusp edge portions 708 by the connecting suture line 704.

To form the connecting suture line 704, one or more first sutures (referred to herein as a first thicker suture or a first main suture) 710 can be paced along the cusp edge portions 708, on one side of the leaflets 702 (FIGS. 22 and 23). In some examples, one or more second sutures (referred to herein as a second thicker suture or a second main suture) 712 can be placed along the cusp edge portions 708, on an opposite side of the leaflets 702.

In some examples, the same first thicker suture 710 and second thicker suture 712 can extend along the cusp edge portions 708 of each and every one of the leaflets 702 (e.g., three shown in FIG. 22) of the leaflet assembly 700, thereby connecting the leaflets 702 together. In other words, a single first thicker suture 710 can extend continuously along the cusp edge portions of all leaflets 702 on one side of the leaflets and a single second thicker suture 712 can extend continuously along the cusp edge portions of all leaflets 702 on the opposite side of the leaflets.

In some examples, each leaflet 702 can include two thicker sutures 710 and 712 (or two thicker suture strands or portions of the same thicker suture) extending along its cusp edge portion 708, on each side of the leaflet 702, and the two thicker sutures 710 and 712 may or may not connect to the two thicker sutures 710 and 712 of the other leaflets 702 of the leaflet assembly 700.

In some examples, the leaflet assembly 700 can be formed with one main or thicker suture (e.g., second suture 712) extending along a first side (e.g., the inner or rougher side 720) of the cusp edge portion 708 of all three leaflets 702 and three separate (or individual) first thicker sutures 710, each extending along a second side (e.g., the outer or smoother side 722) of the cusp edge portion 708 of a corresponding leaflet 702, thereby resulting in two suture end portions (also referred to as suture tails or suture segments) on either end of the cusp edge portion 708 of the leaflet that can extend beyond the commissure tabs 706. As a result, each pair of adjacent suture end portions (or tails) from adjacent leaflets 702 can be joined to form an integral wedge member during commissure assembly (similar to as introduced above with reference to FIG. 17).

In some examples, the leaflet assembly 700 can be formed with three individual first thicker sutures 710, each extending along both the first side and second side of the cusp edge portion 708 of a corresponding leaflet 702. For example, for each leaflet 702, one first thicker suture 710 can be folded over (e.g., in half over) one end of the cusp edge portion 708 and then the resulting first and second suture portions can extend along the first and second sides, respectively, of the cusp edge portion 708 and past an opposite end of the cusp edge portion 708 such that two suture tails or segments are formed at the opposite end of the cusp edge portion 708.

To further form the connecting suture line 704 and connect the first thicker suture(s) 710 and/or the second thicker suture(s) 712 to the leaflets 702, a stitching suture (or third suture) 714 is stitched in a running-stitch pattern (e.g., an in-and-out pattern) through the first thicker suture 710 and/or the second thicker suture 712 and the leaflets 702 (e.g., through the thickness of the tissue of the leaflets 702). For example, if a leaflet 702 includes the first thicker suture 710 (or suture strand or portion) on its first side and the second thicker suture 712 (or suture strand or portion) on its second side, the stitching suture 714 can secure both the first thicker suture 710 and the second thicker suture 712 to the cusp edge portion 708 of the leaflet 702. The stitching suture 714 can form a plurality of in-and-out stitches 715, each of which can extend through the first suture 710, the cusp edge portion 708 of a leaflet 702, and the second suture 712.

In certain examples, the stitching suture 714 is thinner than the suture(s) 710 and/or the suture(s) 712 to facilitate stitching of the stitching suture 714 through sutures 710, 712. Thus, the stitching suture 714 can be referred to as "the thinner suture" in the description below. In such examples, the stitching suture 714 has a smaller diameter and higher gauge than sutures 710, 712. However, it should be noted that in some examples, the stitching suture 714 need not be thinner than sutures 710, 712. In such examples, the stitching suture 714 can have the same diameter (and gauge) as the sutures 710, 712 or a greater diameter (and lower gauge) than sutures 710, 712.

FIG. 23 shows a view of one leaflet 702 with the thinner suture 714 being stitched in the running-stitch pattern through the first thicker suture 710, the cusp edge portion 708 of the leaflet 702, and the second thicker suture 712 (positioned behind the leaflet 702 in the view of FIG. 23), thereby coupling the first thicker suture 710 and the second thicker suture 712 (or two suture portions of the same suture, as described above) to the leaflet 702 and creating the connecting suture line 704.

Thus, the leaflet assembly 700, which includes the leaflets 702 (e.g., three leaflets) assembled together with the connecting suture line 704, can be formed off the frame of the prosthetic heart valve, in a flat configuration (as shown in FIG. 22). By assembling the leaflet assembly 700 off the frame in a flat configuration, the leaflet assembly 700 can be formed more easily and quickly. For example, this can allow the connecting suture line 704 to be sewn automatically (e.g., with sewing machinery) across the flat leaflets 702. Advantageously, this can significantly reduce manufacturing time and costs.

After forming the leaflet assembly 700, the leaflet assembly 700 can be attached to an inner surface of the frame of the prosthetic heart valve (e.g., frame 102 shown in FIGS. 1-3). For example, as shown in FIGS. 24-26, a plurality of stitches 730 (e.g., of a "tucking" suture) can extend underneath the in-and-out stitches 715 (between the in-and-out stitches 715 and the second thicker suture 712) and then loop around angled struts of the frame (e.g., angled struts 130, 132, or 134), thereby connecting the connecting suture line 704 of the cusp edge portion 708 of the leaflets 702 to the angled struts of the frame 102 (FIGS. 24-26).

In some instances, the stitches 730 can extending underneath the second thicker suture 712, between the second thicker suture 712 and the leaflets 702) and then loop around angled struts of the frame.

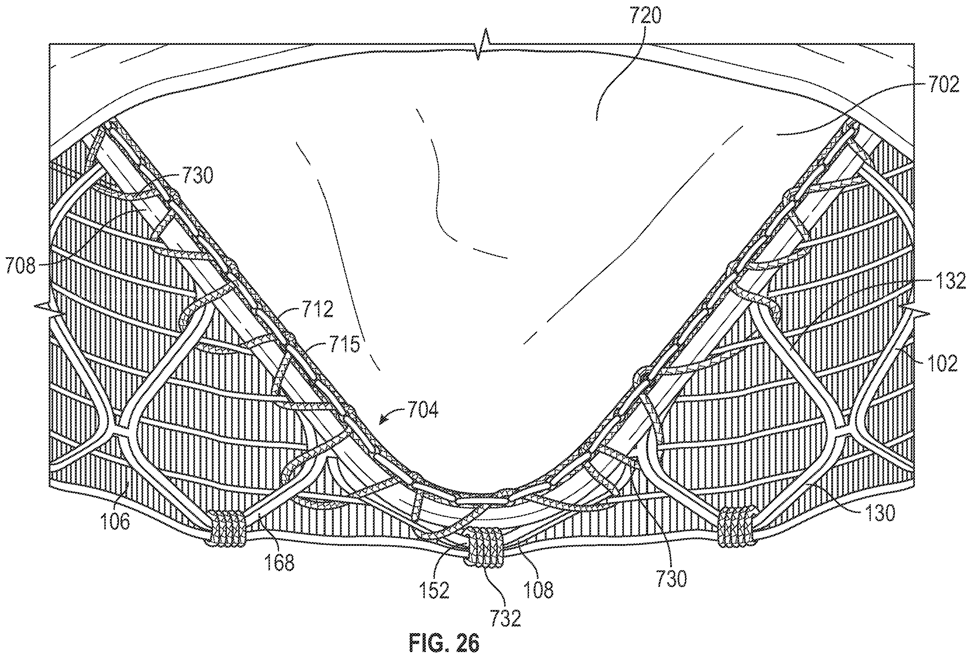

In some examples, as shown in FIGS. 24 and 26, the stitches 730 can also extend through the outer skirt 106 of the prosthetic valve, thereby tightly securing the cusp edge portions 708 of the leaflets 702 to the angled struts and the outer skirt 106.

In some examples, additional stitches 732 can be used to secure an inflow edge 734 of the outer skirt 106 to the inflow struts 168 (such as to the apex regions 152 of the inflow struts 168) (FIGS. 25 and 26).

Further details on connecting suture lines, such as the connecting suture line 704, can be found in U.S. Provisional Application No. 63/343,359.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) is introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (e.g., with heat, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

### Additional Examples of the Disclosed Technology

This application further discloses the additional examples listed below.

A method comprising: forming a stitching line along an edge portion of an outer skirt for a prosthetic heart valve with a plurality of in-and-out stitches that extend along the edge portion, spaced away from a free edge of the edge portion, and a plurality of loops that are spaced apart from one another in a direction of the in-and-out stitches, wherein each loop circles around the free edge of the edge portion and extends between the edge and an intersection of two adjacent in-and-out stitches; and attaching the edge portion to struts of a circumferentially extending row of struts of an annular frame of the prosthetic heart valve by extending a plurality of whip stitches through the plurality of loops and around the struts such that the free edge of the edge portion is positioned against radially outward facing surfaces of the struts and away from leaflets arranged on an inside of the annular frame.

The method of any example herein, wherein loops of the plurality of loops are spaced apart from one another in a direction that is parallel to the free edge.

The method of any example herein, wherein the plurality of in-and-out stitches extend, end-to-end, along the edge portion in parallel to the free edge, and wherein the plurality of in-and-out stitches are spaced away from the free edge by a distance that is perpendicular to the free edge.

The method of any example herein, wherein the distance is in a range of 0.5 - 2.0 mm.

The method of any example herein, wherein the distance is in a range of 1.4 - 2.1 mm.

The method of any example herein, wherein the distance is equal to a length of each loop of the plurality of loops.

The method of any example herein, wherein the plurality of in-and-out stitches are a first pass of first in-and-out stitches formed along the edge portion, wherein each first in-and-out stitch extends between two adjacent apertures of a row of spaced apart apertures in the outer skirt, and wherein forming the stitching line further comprises forming a second pass of second in-and-out stitches along the edge portion that alternate with the first in-and-out stitches such that first and second in-and-out stitches extend across opposing surfaces of the outer skirt between each corresponding pair of two adjacent apertures.

The method of any example herein, wherein the row of spaced apart apertures comprise pre-formed apertures that extend through the outer skirt, between opposing first and second surfaces of the outer skirt.

The method of any example herein, wherein forming the stitching line includes forming the plurality of in-and-out stitches and plurality of loops by: extending a suture, in a direction that is parallel to the free edge, over a first surface of the outer skirt, from a first aperture in the outer skirt to an adjacent, second aperture, thereby forming a first in-and-out stitch across the first surface; extending the suture through the second aperture, across a second surface of the outer skirt that is opposite the first surface toward the free edge, over the free edge, and back across the first surface to the second aperture, thereby forming a first loop; and passing the suture back through the second aperture and across the second surface to an adjacent, third aperture in the outer skirt, thereby forming a second in-and-out stitch across the second surface, and wherein the method for forming the stitching line is repeated in an alternating fashion across the first and second surfaces of the outer skirt to form the plurality of in-and-out stitches and plurality of loops of the stitching line.

The method of any example herein, wherein forming the stitching line includes forming the plurality of in-and-out stitches and plurality of loops by: extending a suture, in a direction that is parallel to the free edge, over a first surface of the outer skirt and through the outer skirt to a second surface of the outer skirt that is opposite the first surface, thereby forming a first in-and-out stitch across the first surface; extending the suture across the second surface toward the free edge, over the free edge, and back across the first surface to an end of the first in-and-out stitch, thereby forming a first loop; and passing the suture back through the outer skirt and across the second surface and through the outer skirt to the first surface, thereby forming a second in-and-out stitch across the second surface, and wherein the method for forming the stitching line is repeated in an alternating fashion across the first and second surfaces of the outer skirt to form the plurality of in-and-out stitches and plurality of loops of the stitching line.

The method of any example herein, wherein attaching the edge portion to the struts by extending the plurality of whip stitches through the plurality of loops and around the struts includes extending at least a portion of the whip stitches around each strut through a corresponding loop of the plurality of loops and around the corresponding loop such that a double-loop is formed around the corresponding loop.

The method of any example herein, wherein the edge portion is an outflow edge portion disposed opposite an inflow edge portion of the outer skirt, wherein the circumferentially extending row of struts that the outflow edge portion is attached to is a first circumferentially extending row of struts of the frame, and wherein the method further comprises securing the inflow edge portion of the outer skirt to a second circumferentially extending row of struts of the frame that defines an inflow end of the frame and is spaced axially away from the first circumferentially extending row of struts of the frame.

The method of any example herein, wherein the first circumferentially extending row of struts of the frame is disposed adjacent to a third circumferentially extending row of struts of the frame that defines an outflow end of the frame.

The method of any example herein, wherein the edge portion extends in a circumferential direction when the outer skirt is disposed around an outer surface of the frame.

The method of any example herein, wherein the edge portion comprises a plurality of projections that define an undulating shape of the edge portion that follows a contour of the struts of circumferentially extending row of struts.

A method comprising: forming a stitching line along an edge portion of an outer skirt for a prosthetic heart valve with a plurality of in-and-out stitches that extend along the edge portion, spaced away from a free edge of the edge portion, and a plurality of pairs of loops that are spaced apart from one another in a direction of the in-and-out stitches, wherein each pair of loops includes a first loop and a second loop, the first loop circling around the free edge of the edge portion and extending between the free edge and the second loop, and the second loop extending between the first loop and an intersection of two adjacent in-and-out stitches of the plurality of in-and-out stitches; and attaching the edge portion to struts of a circumferentially extending row of struts of an annular frame of the prosthetic heart valve by extending whip stitches through the first loop of each pair of loops and around the struts such that the free edge of the edge portion is positioned against radially outward facing surfaces of the struts and away from leaflets arranged on an inside of the annular frame.

The method of any example herein, wherein the plurality of in-and-out stitches extend along the edge portion in parallel to the free edge, and wherein the plurality of in-and-out stitches are spaced away from the free edge by a distance that is perpendicular to the free edge.

The method of any example herein, wherein the distance is in a range of 0.5 - 2.0 mm.

The method of any example herein, wherein the distance is in a range of 1.4 - 2.1 mm.

The method of any example herein, wherein the distance is equal to a combined length of the first loop and the second loop of each pair of loops of the plurality of pairs of loops.

The method of any example herein, wherein the plurality of in-and-out stitches are a first pass of first in-and-out stitches formed along the edge portion, wherein each first in-and-out stitch extends between two adjacent apertures of a row of spaced apart apertures in the outer skirt, and wherein forming the stitching line further comprises forming a second pass of second in-and-out stitches along the edge portion that alternate with the first in-and-out stitches such that first and second in-and-out stitches extend across opposing surfaces of the outer skirt between each corresponding pair of two adjacent apertures.

The method of any example herein, wherein the row of spaced apart apertures comprises pre-formed apertures that extend through the outer skirt, between opposing first and second surfaces of the outer skirt.

The method of any example herein, wherein forming the stitching line includes forming the plurality of in-and-out stitches and the plurality of pairs of loops by: extending a suture, in a direction that is parallel to the free edge, over a first surface of the outer skirt, from a first aperture in the outer skirt to an adjacent second aperture, thereby forming a first in-and-out stitch across the first surface; extending the suture through the second aperture, across a second surface of the outer skirt that is opposite the first surface toward the free edge, through a third aperture that is offset from the second aperture toward the free edge, across the first surface and toward the free edge, over the free edge, back across the second surface to the third aperture, through the third aperture, and back across the first surface to the second aperture, thereby forming the first loop and the second loop of a corresponding pair of loops of the plurality of pairs of loops between the free edge and the in-and-out stitches; and passing the suture back through the second aperture and across the second surface to an adjacent, fourth aperture in the outer skirt, thereby forming a second in-and-out stitch across the second surface, and wherein the method for forming the stitching line is repeated in an alternating fashion across the first and second surfaces of the outer skirt to form the plurality of in-and-out stitches and the plurality of pairs of loops of the stitching line.

The method of any example herein, wherein at least a portion of the first, second, third, and fourth apertures are pre-formed apertures in the outer skirt.

The method of any example herein, wherein at least a portion of the first, second, third, and fourth apertures are not pre-formed apertures and are formed with a needle as the needle passes the suture through the outer skirt, between the first and second surfaces.

The method of any example herein, wherein the first, second, third, and fourth apertures are pre-formed apertures in the outer skirt.

The method of any example herein, wherein forming the stitching line includes forming the plurality of in-and-out stitches and the plurality of pairs of loops by: extending a suture, in a direction that is parallel to the free edge, over a first surface of the outer skirt, from a first point in the outer skirt to an adjacent second point in the outer skirt, thereby forming a first in-and-out stitch across the first surface; extending the suture through the outer skirt at the second point, across a second surface of the outer skirt that is opposite the first surface toward the free edge, through a third point in the outer skirt that is offset from the second point toward the free edge, across the first surface and toward the free edge, over the free edge, back across the second surface to the third point, through the outer skirt at the third point, and back across the first surface to the second point, thereby forming the first loop and the second loop of a corresponding pair of loops of the plurality of pairs of loops between the free edge and the in-and-out stitches; and passing the suture back through the outer skirt at the second point and across the second surface to an adjacent, fourth point in the outer skirt, thereby forming a second in-and-out stitch across the second surface, and wherein the method for forming the stitching line is repeated in an alternating fashion across the first and second surfaces of the outer skirt to form the plurality of in-and-out stitches and the plurality of pairs of loops of the stitching line.

The method of any example herein, wherein each second loop is shorter than each first loop.

The method of any example herein, wherein each first loop is in a range of 0.2 - 0.6 mm.

The method of any example herein, wherein each second loop is in a range of 0.8 - 1.9 mm.

The method of any example herein, wherein the edge portion is an outflow edge portion disposed opposite an inflow edge portion of the outer skirt, wherein the circumferentially extending row of struts that the outflow edge portion is attached to is a first circumferentially extending row of struts of the frame, and wherein the method further comprises securing the inflow edge portion of the outer skirt to a second circumferentially extending row of struts of the frame that defines an inflow end of the frame and is spaced axially away from the first circumferentially extending row of struts of the frame.

The method of any example herein, wherein the first circumferentially extending row of struts of the frame is disposed adjacent to a third circumferentially extending row of struts of the frame that defines an outflow end of the frame.

The method of any example herein, wherein the edge portion extends in a circumferential direction when the outer skirt is disposed around an outer surface of the frame.

The method of any example herein, wherein the edge portion comprises a plurality of projections that define an undulating shape of the edge portion that follows a contour of the struts of circumferentially extending row of struts.

The method of any example herein, wherein attaching the edge portion to the struts by extending the plurality of whip stitches through the plurality of loops and around the struts includes extending at least a portion of the whip stitches around each strut through a corresponding first loop of the plurality of pairs of loops and around the corresponding first loop such that a double-loop is formed around the corresponding first loop.

A prosthetic heart valve comprising: an annular frame comprising a plurality of interconnected struts arranged into a plurality of circumferentially extending rows of struts including a first circumferentially extending row of struts that are disposed closer to an outflow end than an inflow end of the frame; an outer skirt disposed around an outer surface of the frame and comprising an outflow edge portion with a free outflow edge, wherein the outflow edge portion extends circumferentially around the frame and includes a stitching line comprising: a plurality of in-and-out stitches extending along the outflow edge portion in parallel to but spaced away from the outflow edge; and a plurality of looped stitches that are spaced apart from one another in a direction of the plurality of in-and-out stitches, wherein each looped stitch loops around the outflow edge and extends between the outflow edge and an intersection of a corresponding two adjacent in-and-out stitches of the plurality of in-and-out stitches; and a plurality of whip stitches extending through the plurality of looped stitches and around struts of the first circumferentially extending row of struts such that the outflow edge is attached to the struts, against radially outward facing surfaces of the struts, preferably wherein the outflow edge is rougher than a remaining portion of the outflow edge portion of the outer skirt and/or the heart valve further comprising a plurality of leaflets secured inside the frame, and wherein the plurality of leaflets faces radially inward facing surfaces of the struts.

The prosthetic heart valve of any example herein, wherein plurality of circumferentially extending rows of struts further includes a second circumferentially extending row of struts that defines the outflow end of the frame, and wherein the first circumferentially extending row of struts is disposed adjacent to the second circumferentially extending row of struts.

The prosthetic heart valve of any example herein, wherein the outer skirt comprises a row of spaced apart apertures extending along the outflow edge portion in parallel to but spaced away from the outflow edge, and wherein the plurality of in-and-out stitches and the plurality of looped stitches extend through the spaced apart apertures.

The prosthetic heart valve of any example herein, wherein the spaced apart apertures are pre-formed in the outer skirt.

The prosthetic heart valve of any example herein, wherein the plurality of in-and-out stitches comprises a first pass of first in-and-out stitches that extend along the outflow edge portion, and wherein each first in-and-out stitch extends between a corresponding pair of adjacent apertures of the row of spaced apart apertures.

The prosthetic heart valve of any example herein, wherein the plurality of in-and-out stitches further comprises a second pass of second in-and-out stitches that extend along the outflow edge portion, wherein each second in-and-out stitch extends between a corresponding pair of adjacent apertures, and wherein the second in-and-out stitches alternate with the first in-and-out stitches such that first and second in-and-out stitches extend across opposing surfaces of the outer skirt between each corresponding pair of adjacent apertures.

The prosthetic heart valve of any example herein, wherein looped stitches of the plurality of looped stitches are spaced apart from one another in a direction that is parallel to the outflow edge.

The prosthetic heart valve of any example herein, wherein the plurality of in-and-out stitches are spaced away from the outflow edge by a distance that extends perpendicular to the outflow edge.

The prosthetic heart valve of any example herein, wherein the distance is in a range of 0.5 - 2.0 mm.

The prosthetic heart valve of any example herein, wherein the distance is in a range of 1.4 - 2.1 mm.

The prosthetic heart valve of any example herein, wherein the distance is equal to a length of each looped stitch of the plurality of looped stitches.

The prosthetic heart valve of any example herein, wherein each looped stitch has a first portion that extends along a first surface of the outer skirt and a second portion that extends along an opposing, second surface of the outer skirt, between the outflow edge and the intersection of the corresponding two adjacent in-and-out stitches.

The prosthetic heart valve of any example herein, wherein for each strut of the first circumferentially extending row of struts, at least one whip stitch of the plurality of whip stitches extending around the strut forms a double-loop around a corresponding looped stitch.

The prosthetic heart valve of any example herein, wherein the outer skirt comprises an inflow edge portion disposed opposite the outflow edge portion, and wherein the inflow edge portion is secured to a third circumferentially extending row of struts of the plurality of circumferentially extending rows of struts that define the inflow end of the frame.

The prosthetic heart valve of any example herein, wherein the outflow edge portion extends in a circumferential direction around the outer surface of the frame.

The prosthetic heart valve of any example herein, wherein the outflow edge portion comprises a plurality of projections that define an undulating shape of the outflow edge portion that follows a contour of the first circumferentially extending row of struts.

The features described herein with regard to any example can be combined with other features described in any one or more of the other examples, unless otherwise stated. For example, any one or more of the features of one skirt can be combined with any one or more features of another skirt. As another example, any one or more features of one prosthetic heart valve or device can be combined with any one or more features of another prosthetic heart valve or device.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims.

## Claims

1. A method comprising:
forming a stitching line (402, 404, 502, 504, 612, 614) along an edge portion (302, 304, 602, 708) of an outer skirt (106, 300, 652) for a prosthetic heart valve (100, 250) with a plurality of in-and-out stitches (406, 408, 414, 416, 506, 508, 514, 516, 616, 618, 628, 715) that extend along the edge portion (302, 304, 602, 708), spaced away from a free edge of the edge portion (302, 304, 602, 708), and a plurality of loops (410, 510, 512, 528, 620) that are spaced apart from one another in a direction of the in-and-out stitches (406, 408, 414, 416, 506, 508, 514, 516, 616, 618, 628, 715), wherein each loop circles around the free edge of the edge portion (302, 304, 602, 708) and extends between the free edge and an intersection of two adjacent in-and-out stitches (406, 408, 414, 416, 506, 508, 514, 516, 616, 618, 628, 715); and
attaching the edge portion (302, 304, 602, 708) to struts (130, 132, 134, 136, 138, 140, 166, 168, 182, 184, 186, 422, 552, 562, 564, 646) of a circumferentially extending row of struts (130, 132, 134, 136, 138, 140, 166, 168, 182, 184, 186, 422, 552, 562, 564, 646) of an annular frame of the prosthetic heart valve (100, 250) by extending a plurality of whip stitches (420, 522, 560, 632) through the plurality of loops (410, 510, 512, 528, 620) and around the struts (130, 132, 134, 136, 138, 140, 166, 168, 182, 184, 186, 422, 552, 562, 564, 646) such that the free edge of the edge portion (302, 304, 602, 708) is positioned against radially outward facing surfaces of the struts (130, 132, 134, 136, 138, 140, 166, 168, 182, 184, 186, 422, 552, 562, 564, 646) and away from leaflets (112, 600, 702) arranged on an inside of the annular frame.

2. The method of claim 1, wherein the plurality of in-and-out stitches (406, 408, 414, 416, 506, 508, 514, 516, 616, 618, 628, 715) extend, end-to-end, along the edge portion (302, 304, 602, 708) in parallel to the free edge, and wherein the plurality of in-and-out stitches (406, 408, 414, 416, 506, 508, 514, 516, 616, 618, 628, 715) are spaced away from the free edge by a distance (418, 518, 630) that is perpendicular to the free edge, preferably wherein the distance (418, 518, 630) is in a range of 0.5 - 2.0 mm.

3. The method of any one of claims 1 or 2, wherein the plurality of in-and-out stitches (406, 408, 414, 416, 506, 508, 514, 516, 616, 618, 628, 715) are a first pass of first in-and-out stitches (406, 408, 414, 416, 506, 508, 514, 516, 616, 618, 628, 715) formed along the edge portion (302, 304, 602, 708), wherein each first in-and-out stitch extends between two adjacent apertures of a row of spaced apart apertures in the outer skirt (106, 300, 652), and wherein forming the stitching line (402, 404, 502, 504, 612, 614) further comprises forming a second pass of second in-and-out stitches (406, 408, 414, 416, 506, 508, 514, 516, 616, 618, 628, 715) along the edge portion (302, 304, 602, 708) that alternate with the first in-and-out stitches (406, 408, 414, 416, 506, 508, 514, 516, 616, 618, 628, 715) such that first and second in-and-out stitches (406, 408, 414, 416, 506, 508, 514, 516, 616, 618, 628, 715) extend across opposing surfaces of the outer skirt (106, 300, 652) between each corresponding pair of two adjacent apertures.

4. The method of any one of claims 1-3, wherein forming the stitching line (402, 404, 502, 504, 612, 614) includes forming the plurality of in-and-out stitches (406, 408, 414, 416, 506, 508, 514, 516, 616, 618, 628, 715) and plurality of loops (410, 510, 512, 528, 620) by:
extending a suture (412, 513, 526, 622, 636, 710, 712, 714), in a direction that is parallel to the free edge, over a first surface (322) of the outer skirt (106, 300, 652) and through the outer skirt (106, 300, 652) to a second surface (324) of the outer skirt (106, 300, 652) that is opposite the first surface (322), thereby forming a first in-and-out stitch across the first surface (322);
extending the suture (412, 513, 526, 622, 636, 710, 712, 714) across the second surface (324) toward the free edge, over the free edge, and back across the first surface (322) to an end of the first in-and-out stitch, thereby forming a first loop (510); and
passing the suture (412, 513, 526, 622, 636, 710, 712, 714) back through the outer skirt (106, 300, 652) and across the second surface (324) and through the outer skirt (106, 300, 652) to the first surface (322), thereby forming a second in-and-out stitch across the second surface (324), and wherein the method for forming the stitching line (402, 404, 502, 504, 612, 614) is repeated in an alternating fashion across the first and second surfaces (324) of the outer skirt (106, 300, 652) to form the plurality of in-and-out stitches (406, 408, 414, 416, 506, 508, 514, 516, 616, 618, 628, 715) and plurality of loops (410, 510, 512, 528, 620) of the stitching line (402, 404, 502, 504, 612, 614).

5. The method of any one of claims 1-4, wherein attaching the edge portion (302, 304, 602, 708) to the struts (130, 132, 134, 136, 138, 140, 166, 168, 182, 184, 186, 422, 552, 562, 564, 646) by extending the plurality of whip stitches (420, 522, 560, 632) through the plurality of loops (410, 510, 512, 528, 620) and around the struts (130, 132, 134, 136, 138, 140, 166, 168, 182, 184, 186, 422, 552, 562, 564, 646) includes extending at least a portion (146, 148, 150, 156, 210, 224, 228, 302, 304, 602, 644, 708) of the whip stitches (420, 522, 560, 632) around each strut (130, 132, 134, 136, 138, 140, 166, 168, 182, 184, 186, 422, 552, 562, 564, 646) through a corresponding loop of the plurality of loops (410, 510, 512, 528, 620) and around the corresponding loop such that a double-loop (528) is formed around the corresponding loop.

6. The method of any one of claims 1-5, wherein the edge portion (302, 304, 602, 708) is an outflow edge portion (306) disposed opposite an inflow edge portion (308) of the outer skirt (106, 300, 652), wherein the circumferentially extending row of struts (130, 132, 134, 136, 138, 140, 166, 168, 182, 184, 186, 422, 552, 562, 564, 646) that the outflow edge portion (306) is attached to is a first circumferentially extending row of struts (130, 132, 134, 136, 138, 140, 166, 168, 182, 184, 186, 422, 552, 562, 564, 646) of the frame (102, 550, 650), and wherein the method further comprises securing the inflow edge portion (308) of the outer skirt (106, 300, 652) to a second circumferentially extending row of struts (130, 132, 134, 136, 138, 140, 166, 168, 182, 184, 186, 422, 552, 562, 564, 646) of the frame (102, 550, 650) that defines an inflow end (108, 648) of the frame (102, 550, 650) and is spaced axially away from the first circumferentially extending row of struts (130, 132, 134, 136, 138, 140, 166, 168, 182, 184, 186, 422, 552, 562, 564, 646) of the frame (102, 550, 650), preferably wherein the first circumferentially extending row of struts (130, 132, 134, 136, 138, 140, 166, 168, 182, 184, 186, 422, 552, 562, 564, 646) of the frame (102, 550, 650) is disposed adjacent to a third circumferentially extending row of struts (130, 132, 134, 136, 138, 140, 166, 168, 182, 184, 186, 422, 552, 562, 564, 646) of the frame (102, 550, 650) that defines an outflow end (110) of the frame (102, 550, 650).

7. A prosthetic heart valve (100, 250) comprising:
an annular frame comprising a plurality of interconnected struts (116) arranged into a plurality of circumferentially extending rows of struts (130, 132, 134, 136, 138, 140, 166, 168, 182, 184, 186, 422, 552, 562, 564, 646) including a first circumferentially extending row of struts (130, 132, 134, 136, 138, 140, 166, 168, 182, 184, 186, 422, 552, 562, 564, 646) that is disposed closer to an outflow end (110) than an inflow end (108, 648) of the frame (102, 550, 650);
an outer skirt (106, 300, 652) disposed around an outer surface (172) of the frame (102, 550, 650) and comprising an outflow edge portion (306) with a free outflow edge, wherein the outflow edge portion (306) extends circumferentially around the frame (102, 550, 650) and includes a stitching line (402, 404, 502, 504, 612, 614) comprising:
a plurality of in-and-out stitches (406, 408, 414, 416, 506, 508, 514, 516, 616, 618, 628, 715) extending along the outflow edge portion (306) in parallel to but spaced away from the outflow edge (320, 604); and
a plurality of looped stitches that are spaced apart from one another in a direction of the plurality of in-and-out stitches (406, 408, 414, 416, 506, 508, 514, 516, 616, 618, 628, 715), wherein each looped stitch loops (410, 510, 512, 528, 620) around the outflow edge (320, 604) and extends between the outflow edge (320, 604) and an intersection of corresponding two adjacent in-and-out stitches (406, 408, 414, 416, 506, 508, 514, 516, 616, 618, 628, 715) of the plurality of in-and-out stitches (406, 408, 414, 416, 506, 508, 514, 516, 616, 618, 628, 715); and
a plurality of whip stitches (420, 522, 560, 632) extending through the plurality of looped stitches and around struts (130, 132, 134, 136, 138, 140, 166, 168, 182, 184, 186, 422, 552, 562, 564, 646) of the first circumferentially extending row of struts (130, 132, 134, 136, 138, 140, 166, 168, 182, 184, 186, 422, 552, 562, 564, 646) such that the outflow edge (320, 604) is attached to the struts (130, 132, 134, 136, 138, 140, 166, 168, 182, 184, 186, 422, 552, 562, 564, 646), against radially outward facing surfaces of the struts (130, 132, 134, 136, 138, 140, 166, 168, 182, 184, 186, 422, 552, 562, 564, 646), preferably wherein
the outflow edge (320, 604) is rougher than a remaining portion of the outflow edge portion (306) of the outer skirt (106, 300, 652),
and/or
the heart valve further comprises a plurality of leaflets (112, 600, 702) secured inside the frame (102, 550, 650), and wherein the plurality of leaflets (112, 600, 702) faces radially inward facing surfaces of the struts (130, 132, 134, 136, 138, 140, 166, 168, 182, 184, 186, 422, 552, 562, 564, 646).

8. The prosthetic heart valve (100, 250) of claim 7, wherein plurality of circumferentially extending rows of struts (130, 132, 134, 136, 138, 140, 166, 168, 182, 184, 186, 422, 552, 562, 564, 646) further includes a second circumferentially extending row of struts (130, 132, 134, 136, 138, 140, 166, 168, 182, 184, 186, 422, 552, 562, 564, 646) that defines the outflow end (110) of the frame (102, 550, 650), and wherein the first circumferentially extending row of struts (130, 132, 134, 136, 138, 140, 166, 168, 182, 184, 186, 422, 552, 562, 564, 646) is disposed adjacent to the second circumferentially extending row of struts (130, 132, 134, 136, 138, 140, 166, 168, 182, 184, 186, 422, 552, 562, 564, 646).

9. The prosthetic heart valve (100, 250) of any one of claims 7 or 8, wherein the outer skirt (106, 300, 652) comprises a row of spaced apart apertures extending along the outflow edge portion (306) in parallel to but spaced away from the outflow edge (320, 604), and wherein the plurality of in-and-out stitches (406, 408, 414, 416, 506, 508, 514, 516, 616, 618, 628, 715) and the plurality of looped stitches extend through the spaced apart apertures of the row of spaced apart apertures.

10. The prosthetic heart valve (100, 250) of any one of claims 7-9, wherein the plurality of in-and-out stitches (406, 408, 414, 416, 506, 508, 514, 516, 616, 618, 628, 715) are spaced away from the outflow edge (320, 604) by a distance (418, 518, 630) that extends perpendicular to the outflow edge (320, 604), preferably wherein the distance (418, 518, 630) is in a range of 0.5 - 2.0 mm.

11. The prosthetic heart valve (100, 250) of any one of claims 7-10, wherein the outflow edge portion (306) comprises a plurality of projections (312) that define an undulating shape of the outflow edge portion (306) that follows a contour of the first circumferentially extending row of struts (130, 132, 134, 136, 138, 140, 166, 168, 182, 184, 186, 422, 552, 562, 564, 646).

12. A method comprising:
forming a stitching line (402, 404, 502, 504, 612, 614) along a cusp edge portion (602, 708) of a leaflet (112, 600, 702) for a prosthetic heart valve (100, 250) with a plurality of in-and-out stitches (406, 408, 414, 416, 506, 508, 514, 516, 616, 618, 628, 715) that extend along the cusp edge portion (602, 708), spaced away from an outermost cusp edge (608) of the cusp edge portion (602, 708), and a plurality of loops (410, 510, 512, 528, 620) that are spaced apart from one another in a direction of the in-and-out stitches (406, 408, 414, 416, 506, 508, 514, 516, 616, 618, 628, 715), wherein each loop circles around the cusp edge (608) of the cusp edge portion (602, 708) and extends between the cusp edge (608) and an intersection of two adjacent in-and-out stitches (406, 408, 414, 416, 506, 508, 514, 516, 616, 618, 628, 715); and
attaching the cusp edge portion (602, 708) to a target component (634) of the prosthetic heart valve (100, 250) by extending a plurality of whip stitches (420, 522, 560, 632) through the plurality of loops (410, 510, 512, 528, 620) and around or through the target component (634) such that the cusp edge (608) of the cusp edge portion (602, 708) is tucked against the target component (634).

13. The method of claim 12, wherein the target component (634) is a fabric skirt configured to be attached to an inner surface of an annular frame of the prosthetic heart valve (100, 250).

14. The method of claim 12, wherein the target component (634) is struts (130, 132, 134, 136, 138, 140, 166, 168, 182, 184, 186, 422, 552, 562, 564, 646) of an annular frame of the prosthetic heart valve (100, 250) that follow a scallop line of the cusp edge portion (602, 708).

15. The method of any one of claims 12-14, wherein the plurality of in-and-out stitches (406, 408, 414, 416, 506, 508, 514, 516, 616, 618, 628, 715) are a first pass of first in-and-out stitches (406, 408, 414, 416, 506, 508, 514, 516, 616, 618, 628, 715) formed along the cusp edge portion (602, 708), and wherein forming the stitching line (402, 404, 502, 504, 612, 614) further comprises forming a second pass of second in-and-out stitches (406, 408, 414, 416, 506, 508, 514, 516, 616, 618, 628, 715) along the cusp edge portion (602, 708) that alternate with the first in-and-out stitches (406, 408, 414, 416, 506, 508, 514, 516, 616, 618, 628, 715) such that first and second in-and-out stitches (406, 408, 414, 416, 506, 508, 514, 516, 616, 618, 628, 715) extend across opposing sides of the leaflet (112, 600, 702) between each pair of adjacent loops (410, 510, 512, 528, 620) of the plurality of loops (410, 510, 512, 528, 620).

## Patentansprüche

1. Verfahren, umfassend:
Bilden einer Nahtlinie (402, 404, 502, 504, 612, 614) entlang eines Randabschnitts (302, 304, 602, 708) einer Außenschürze (106, 300, 652) für eine Prothesenherzklappe (100, 250) mit einer Vielzahl von Ein- und Ausstichen (406, 408, 414, 416, 506, 508, 514, 516, 616, 618, 628, 715), die sich entlang des Randabschnitts (302, 304, 602, 708) erstrecken, beabstandet von einer freien Kante des Randabschnitts (302, 304, 602, 708), und einer Vielzahl von Schlaufen (410, 510, 512, 528, 620), die in einer Richtung der Ein- und Ausstiche (406, 408, 414, 416, 506, 508, 514, 516, 616, 618, 628, 715) voneinander beabstandet sind, wobei jede Schlaufe um die freie Kante des Randabschnitts (302, 304, 602, 708) herumführt und sich zwischen der freien Kante und einem Schnittpunkt zweier benachbarter Ein- und Ausstiche (406, 408, 414, 416, 506, 508, 514, 516, 616, 618, 628, 715) erstreckt; und
Befestigen des Randabschnitts (302, 304, 602, 708) an Streben (130, 132, 134, 136, 138, 140, 166, 168, 182, 184, 186, 422, 552, 562, 564, 646) einer sich in Umfangsrichtung erstreckenden Reihe von Streben (130, 132, 134, 136, 138, 140, 166, 168, 182, 184, 186, 422, 552, 562, 564, 646) eines ringförmigen Rahmens der Prothesenherzklappe (100, 250), indem eine Vielzahl von Überwendlichstichen (420, 522, 560, 632) durch die Vielzahl von Schlaufen (410, 510, 512, 528, 620) und um die Streben (130, 132, 134, 136, 138, 140, 166, 168, 182, 184, 186, 422, 552, 562, 564, 646) herumgeführt wird, so dass die freie Kante des Randabschnitts (302, 304, 602, 708) gegen radial nach außen weisende Flächen der Streben (130, 132, 134, 136, 138, 140, 166, 168, 182, 184, 186, 422, 552, 562, 564, 646) und entfernt von Klappensegeln (112, 600, 702), die an einer Innenseite des ringförmigen Rahmens angeordnet sind, positioniert ist.

2. Verfahren nach Anspruch 1, wobei sich die Vielzahl von Ein- und Ausstichen (406, 408, 414, 416, 506, 508, 514, 516, 616, 618, 628, 715) Ende-zu-Ende entlang des Randabschnitts (302, 304, 602, 708) parallel zur freien Kante erstreckt, und wobei die Vielzahl von Ein- und Ausstichen (406, 408, 414, 416, 506, 508, 514, 516, 616, 618, 628, 715) um einen Abstand (418, 518, 630), der sich senkrecht zur freien Kante erstreckt, von der freien Kante beabstandet ist, vorzugsweise wobei der Abstand (418, 518, 630) in einem Bereich von 0,5 - 2,0 mm liegt.

3. Verfahren nach einem der Ansprüche 1 oder 2, wobei die Vielzahl von Ein- und Ausstichen (406, 408, 414, 416, 506, 508, 514, 516, 616, 618, 628, 715) ein erster Durchgang von ersten Ein- und Ausstichen (406, 408, 414, 416, 506, 508, 514, 516, 616, 618, 628, 715) ist, die entlang des Randabschnitts (302, 304, 602, 708) gebildet werden, wobei sich jeder erste Ein- und Ausstich zwischen zwei benachbarten Öffnungen einer Reihe voneinander beabstandeter Öffnungen in der Außenschürze (106, 300, 652) erstreckt, und wobei das Bilden der Nahtlinie (402, 404, 502, 504, 612, 614) ferner das Bilden eines zweiten Durchgangs von zweiten Ein- und Ausstichen (406, 408, 414, 416, 506, 508, 514, 516, 616, 618, 628, 715) entlang des Randabschnitts (302, 304, 602, 708) umfasst, die mit den ersten Ein- und Ausstichen (406, 408, 414, 416, 506, 508, 514, 516, 616, 618, 628, 715) alternieren, so dass sich erste und zweite Ein- und Ausstiche (406, 408, 414, 416, 506, 508, 514, 516, 616, 618, 628, 715) über gegenüberliegende Flächen der Außenschürze (106, 300, 652) zwischen jedem entsprechenden Paar zweier benachbarter Öffnungen erstrecken.

4. Verfahren nach einem der Ansprüche 1 bis 3, wobei das Bilden der Nahtlinie (402, 404, 502, 504, 612, 614) das Bilden der Vielzahl von Ein- und Ausstichen (406, 408, 414, 416, 506, 508, 514, 516, 616, 618, 628, 715) und der Vielzahl von Schlaufen (410, 510, 512, 528, 620) umfasst durch:
Führen eines Fadens (412, 513, 526, 622, 636, 710, 712, 714) in einer Richtung, die sich parallel zur freien Kante erstreckt, über eine erste Fläche (322) der Außenschürze (106, 300, 652) und durch die Außenschürze (106, 300, 652) zu einer zweiten Fläche (324) der Außenschürze (106, 300, 652), die der ersten Fläche (322) gegenüberliegt, wodurch ein erster Ein- und Ausstich über die erste Fläche (322) gebildet wird;
Führen des Fadens (412, 513, 526, 622, 636, 710, 712, 714) über die zweite Fläche (324) in Richtung der freien Kante, über die freie Kante und zurück über die erste Fläche (322) zu einem Ende des ersten Ein- und Ausstichs, wodurch eine erste Schlaufe (510) gebildet wird; und
Zurückführen des Fadens (412, 513, 526, 622, 636, 710, 712, 714) durch die Außenschürze (106, 300, 652) und über die zweite Fläche (324) und durch die Außenschürze (106, 300, 652) zur ersten Fläche (322), wodurch ein zweiter Ein- und Ausstich über die zweite Fläche (324) gebildet wird, und wobei das Verfahren zum Bilden der Nahtlinie (402, 404, 502, 504, 612, 614) in wechselnder Abfolge über die erste und zweite Fläche (324) der Außenschürze (106, 300, 652) wiederholt wird, um die Vielzahl von Ein- und Ausstichen (406, 408, 414, 416, 506, 508, 514, 516, 616, 618, 628, 715) und die Vielzahl von Schlaufen (410, 510, 512, 528, 620) der Nahtlinie (402, 404, 502, 504, 612, 614) zu bilden.

5. Verfahren nach einem der Ansprüche 1 bis 4, wobei das Befestigen des Randabschnitts (302, 304, 602, 708) an den Streben (130, 132, 134, 136, 138, 140, 166, 168, 182, 184, 186, 422, 552, 562, 564, 646) durch Führen der Vielzahl von Überwendlichstichen (420, 522, 560, 632) durch die Vielzahl von Schlaufen (410, 510, 512, 528, 620) und um die Streben (130, 132, 134, 136, 138, 140, 166, 168, 182, 184, 186, 422, 552, 562, 564, 646) herum das Führen zumindest eines Teils (146, 148, 150, 156, 210, 224, 228, 302, 304, 602, 644, 708) der Überwendlichstiche (420, 522, 560, 632) um jede Strebe (130, 132, 134, 136, 138, 140, 166, 168, 182, 184, 186, 422, 552, 562, 564, 646) durch eine entsprechende Schlaufe der Vielzahl von Schlaufen (410, 510, 512, 528, 620) und um die entsprechende Schlaufe herum umfasst, so dass eine Doppelschlaufe (528) um die entsprechende Schlaufe gebildet wird.

6. Verfahren nach einem der Ansprüche 1 bis 5, wobei der Randabschnitt (302, 304, 602, 708) ein Ausström-Randabschnitt (306) ist, der gegenüber einem Einström-Randabschnitt (308) der Außenschürze (106, 300, 652) angeordnet ist, wobei die sich in Umfangsrichtung erstreckende Reihe von Streben (130, 132, 134, 136, 138, 140, 166, 168, 182, 184, 186, 422, 552, 562, 564, 646), an der der Ausström-Randabschnitt (306) befestigt ist, eine erste sich in Umfangsrichtung erstreckende Reihe von Streben (130, 132, 134, 136, 138, 140, 166, 168, 182, 184, 186, 422, 552, 562, 564, 646) des Rahmens (102, 550, 650) ist, und wobei das Verfahren ferner das Befestigen des Einström-Randabschnitts (308) der Außenschürze (106, 300, 652) an einer zweiten sich in Umfangsrichtung erstreckenden Reihe von Streben (130, 132, 134, 136, 138, 140, 166, 168, 182, 184, 186, 422, 552, 562, 564, 646) des Rahmens (102, 550, 650) umfasst, die ein Einströmende (108, 648) des Rahmens (102, 550, 650) definiert und axial beabstandet von der ersten sich in Umfangsrichtung erstreckenden Reihe von Streben (130, 132, 134, 136, 138, 140, 166, 168, 182, 184, 186, 422, 552, 562, 564, 646) des Rahmens (102, 550, 650) angeordnet ist, vorzugsweise wobei die erste sich in Umfangsrichtung erstreckende Reihe von Streben (130, 132, 134, 136, 138, 140, 166, 168, 182, 184, 186, 422, 552, 562, 564, 646) des Rahmens (102, 550, 650) benachbart zu einer dritten sich in Umfangsrichtung erstreckenden Reihe von Streben (130, 132, 134, 136, 138, 140, 166, 168, 182, 184, 186, 422, 552, 562, 564, 646) des Rahmens (102, 550, 650) angeordnet ist, die ein Ausströmende (110) des Rahmens (102, 550, 650) definiert.

7. Prothesenherzklappe (100, 250), umfassend:
einen ringförmigen Rahmen, der eine Vielzahl von miteinander verbundenen Streben (116) umfasst, die in eine Vielzahl von sich in Umfangsrichtung erstreckenden Reihen von Streben (130, 132, 134, 136, 138, 140, 166, 168, 182, 184, 186, 422, 552, 562, 564, 646) angeordnet sind, einschließlich einer ersten sich in Umfangsrichtung erstreckenden Reihe von Streben (130, 132, 134, 136, 138, 140, 166, 168, 182, 184, 186, 422, 552, 562, 564, 646), die näher an einem Ausströmende (110) als an einem Einströmende (108, 648) des Rahmens (102, 550, 650) angeordnet ist;
eine Außenschürze (106, 300, 652), die um eine Außenfläche (172) des Rahmens (102, 550, 650) angeordnet ist und einen Ausström-Randabschnitt (306) mit einer freien Ausström-Kante umfasst, wobei sich der Ausström-Randabschnitt (306) in Umfangsrichtung um den Rahmen (102, 550, 650) erstreckt und eine Nahtlinie (402, 404, 502, 504, 612, 614) umfasst, die aufweist:
eine Vielzahl von Ein- und Ausstichen (406, 408, 414, 416, 506, 508, 514, 516, 616, 618, 628, 715), die sich entlang des Ausström-Randabschnitts (306) parallel zur, jedoch beabstandet von der Ausström-Kante (320, 604) erstrecken; und
eine Vielzahl von Schlaufenstichen, die in einer Richtung der Vielzahl von Ein- und Ausstichen (406, 408, 414, 416, 506, 508, 514, 516, 616, 618, 628, 715) voneinander beabstandet sind, wobei jeder Schlaufenstich (410, 510, 512, 528, 620) um die Ausström-Kante (320, 604) herumführt und sich zwischen der Ausström-Kante (320, 604) und einem Schnittpunkt zweier benachbarter Ein- und Ausstiche (406, 408, 414, 416, 506, 508, 514, 516, 616, 618, 628, 715) der Vielzahl von Ein- und Ausstichen (406, 408, 414, 416, 506, 508, 514, 516, 616, 618, 628, 715) erstreckt; und
eine Vielzahl von Überwendlichstichen (420, 522, 560, 632), die durch die Vielzahl von Schlaufenstichen und um Streben (130, 132, 134, 136, 138, 140, 166, 168, 182, 184, 186, 422, 552, 562, 564, 646) der ersten sich in Umfangsrichtung erstreckenden Reihe von Streben (130, 132, 134, 136, 138, 140, 166, 168, 182, 184, 186, 422, 552, 562, 564, 646) geführt sind, so dass die Ausström-Kante (320, 604) an den Streben (130, 132, 134, 136, 138, 140, 166, 168, 182, 184, 186, 422, 552, 562, 564, 646) gegen radial nach außen weisende Flächen der Streben befestigt ist, vorzugsweise wobei
die Ausström-Kante (320, 604) rauer ist als ein übriger Abschnitt des Ausström-Randabschnitts (306) der Außenschürze (106, 300, 652), und/oder
die Herzklappe ferner eine Vielzahl von Klappensegeln (112, 600, 702) umfasst, die innerhalb des Rahmens (102, 550, 650) befestigt sind, wobei die Vielzahl von Klappensegeln (112, 600, 702) radial nach innen weisenden Flächen der Streben (130, 132, 134, 136, 138, 140, 166, 168, 182, 184, 186, 422, 552, 562, 564, 646) zugewandt ist.

8. Prothesenherzklappe (100, 250) nach Anspruch 7, wobei die Vielzahl von sich in Umfangsrichtung erstreckenden Reihen von Streben (130, 132, 134, 136, 138, 140, 166, 168, 182, 184, 186, 422, 552, 562, 564, 646) ferner eine zweite sich in Umfangsrichtung erstreckende Reihe von Streben (130, 132, 134, 136, 138, 140, 166, 168, 182, 184, 186, 422, 552, 562, 564, 646) umfasst, die das Ausströmende (110) des Rahmens (102, 550, 650) definiert, und wobei die erste sich in Umfangsrichtung erstreckende Reihe von Streben (130, 132, 134, 136, 138, 140, 166, 168, 182, 184, 186, 422, 552, 562, 564, 646) benachbart zu der zweiten sich in Umfangsrichtung erstreckenden Reihe von Streben angeordnet ist.

9. Prothesenherzklappe (100, 250) nach einem der Ansprüche 7 oder 8, wobei die Außenschürze (106, 300, 652) eine Reihe voneinander beabstandeter Öffnungen umfasst, die sich entlang des Ausström-Randabschnitts (306) parallel zur, jedoch beabstandet von der Ausström-Kante (320, 604) erstreckt, und wobei die Vielzahl von Ein- und Ausstichen (406, 408, 414, 416, 506, 508, 514, 516, 616, 618, 628, 715) und die Vielzahl von Schlaufenstichen durch die beabstandeten Öffnungen der Reihe geführt sind.

10. Prothesenherzklappe (100, 250) nach einem der Ansprüche 7 bis 9, wobei die Vielzahl von Ein- und Ausstichen (406, 408, 414, 416, 506, 508, 514, 516, 616, 618, 628, 715) um einen Abstand (418, 518, 630) von der Ausström-Kante (320, 604) beabstandet ist, der sich senkrecht zur Ausström-Kante (320, 604) erstreckt, vorzugsweise wobei der Abstand (418, 518, 630) in einem Bereich von 0,5 - 2,0 mm liegt.

11. Prothesenherzklappe (100, 250) nach einem der Ansprüche 7 bis 10, wobei der Ausström-Randabschnitt (306) eine Vielzahl von Vorsprüngen (312) umfasst, die eine wellenförmige Gestalt des Ausström-Randabschnitts (306) definieren, die einem Verlauf der ersten sich in Umfangsrichtung erstreckenden Reihe von Streben (130, 132, 134, 136, 138, 140, 166, 168, 182, 184, 186, 422, 552, 562, 564, 646) folgt.

12. Verfahren, umfassend:
Bilden einer Nahtlinie (402, 404, 502, 504, 612, 614) entlang eines Kuspenrandabschnitts (602, 708) eines Klappensegels (112, 600, 702) für eine Prothesenherzklappe (100, 250) mit einer Vielzahl von Ein- und Ausstichen (406, 408, 414, 416, 506, 508, 514, 516, 616, 618, 628, 715), die sich entlang des Kuspenrandabschnitts (602, 708) erstrecken, beabstandet von einer äußersten Kuspenkante (608) des Kuspenrandabschnitts (602, 708), und einer Vielzahl von Schlaufen (410, 510, 512, 528, 620), die in einer Richtung der Ein- und Ausstiche (406, 408, 414, 416, 506, 508, 514, 516, 616, 618, 628, 715) voneinander beabstandet sind, wobei jede Schlaufe um die Kuspenkante (608) des Kuspenrandabschnitts (602, 708) herumführt und sich zwischen der Kuspenkante (608) und einem Schnittpunkt zweier benachbarter Ein- und Ausstiche erstreckt; und
Befestigen des Kuspenrandabschnitts (602, 708) an einer Zielkomponente (634) der Prothesenherzklappe (100, 250), indem eine Vielzahl von Überwendlichstichen (420, 522, 560, 632) durch die Vielzahl von Schlaufen (410, 510, 512, 528, 620) und um oder durch die Zielkomponente (634) herumgeführt wird, so dass die Kuspenkante (608) des Kuspenrandabschnitts (602, 708) an die Zielkomponente (634) angelegt ist.

13. Verfahren nach Anspruch 12, wobei die Zielkomponente (634) eine Stoffschürze ist, die dazu konfiguriert ist, an einer Innenfläche eines ringförmigen Rahmens der Prothesenherzklappe (100, 250) befestigt zu werden.

14. Verfahren nach Anspruch 12, wobei die Zielkomponente (634) Streben (130, 132, 134, 136, 138, 140, 166, 168, 182, 184, 186, 422, 552, 562, 564, 646) eines ringförmigen Rahmens der Prothesenherzklappe (100, 250) sind, die einer Wellenlinie des Kuspenrandabschnitts (602, 708) folgen.

15. Verfahren nach einem der Ansprüche 12 bis 14, wobei die Vielzahl von Ein- und Ausstichen (406, 408, 414, 416, 506, 508, 514, 516, 616, 618, 628, 715) ein erster Durchgang von ersten Ein- und Ausstichen (406, 408, 414, 416, 506, 508, 514, 516, 616, 618, 628, 715) ist, die entlang des Kuspenrandabschnitts (602, 708) gebildet werden, und wobei das Bilden der Nahtlinie (402, 404, 502, 504, 612, 614) ferner das Bilden eines zweiten Durchgangs von zweiten Ein- und Ausstichen (406, 408, 414, 416, 506, 508, 514, 516, 616, 618, 628, 715) entlang des Kuspenrandabschnitts (602, 708) umfasst, die mit den ersten Ein- und Ausstichen alternieren, so dass sich erste und zweite Ein- und Ausstiche über gegenüberliegende Seiten des Klappensegels (112, 600, 702) zwischen jedem Paar benachbarter Schlaufen (410, 510, 512, 528, 620) der Vielzahl von Schlaufen (410, 510, 512, 528, 620) erstrecken.

## Revendications

1. Procédé comprenant :
la formation d'une ligne de couture (402, 404, 502, 504, 612, 614) le long d'une partie de bord (302, 304, 602, 708) d'une collerette externe (106, 300, 652) pour une valvule cardiaque prothétique (100, 250) présentant une pluralité de points d'entrée-sortie (406, 408, 414, 416, 506, 508, 514, 516, 616, 618, 628, 715) qui s'étendent le long de la partie de bord (302, 304, 602, 708), écartés d'un bord libre de la partie de bord (302, 304, 602, 708) et une pluralité de boucles (410, 510, 512, 528, 620) qui sont espacées les unes des autres dans une direction des points d'entrée-sortie (406, 408, 414, 416, 506, 508, 514, 516, 616, 618, 628, 715), chaque boucle tournant autour du bord libre de la partie de bord (302, 304, 602, 708) et s'étendant entre le bord libre et une intersection de deux points d'entrée-sortie (406, 408, 414, 416, 506, 508, 514, 516, 616, 618, 628, 715) adjacents ; et
l'attache de la partie de bord (302, 304, 602, 708) à des entretoises (130, 132, 134, 136, 138, 140, 166, 168, 182, 184, 186, 422, 552, 562, 564, 646) d'une rangée d'entretoises (130, 132, 134, 136, 138, 140, 166, 168 182, 184, 186, 422, 552, 562, 564, 646) s'étendant circonférentiellement d'un cadre annulaire de la valvule cardiaque prothétique (100, 250) par extension d'une pluralité de points de surjet (420, 522, 560, 632) à travers la pluralité de boucles (410, 510, 512, 528, 620) et autour des entretoises (130, 132, 134, 136, 138, 140, 166, 168, 182, 184, 186, 422, 552, 562, 564, 646) de telle sorte que le bord libre de la partie de bord (302, 304, 602, 708) est positionné contre des surfaces orientées radialement vers l'extérieur des entretoises (130, 132, 134, 136, 138, 140, 166, 168, 182, 184, 186, 422, 552, 562, 564, 646) et à l'opposé de feuillets (112, 600, 702) agencés sur un intérieur du cadre annulaire.

2. Procédé selon la revendication 1, la pluralité de points d'entrée-sortie (406, 408, 414, 416, 506, 508, 514, 516, 616, 618, 628, 715) s'étendant, bout à bout, le long de la partie de bord (302, 304, 602, 708) parallèlement au bord libre et la pluralité de points d'entréesortie (406, 408, 414, 416, 506, 508, 514, 516, 616, 618, 628, 715) étant écartés du bord libre d'une distance (418, 518, 630) qui est perpendiculaire au bord libre, de préférence la distance (418, 518, 630) étant située dans une plage de 0,5 à 2,0 mm.

3. Procédé selon l'une quelconque des revendications 1 ou 2, la pluralité de points d'entrée-sortie (406, 408, 414, 416, 506, 508, 514, 516, 616, 618, 628, 715) étant une première passe de premiers points d'entrée-sortie (406, 408, 414, 416, 506, 508, 514, 516, 616, 618, 628, 715) formés le long de la partie de bord (302, 304, 602, 708), chaque premier point d'entrée-sortie s'étendant entre deux ouvertures adjacentes d'une rangée d'ouvertures espacées dans la collerette externe (106, 300, 652) et la formation de la ligne de couture (402, 404, 502, 504, 612, 614) comprenant en outre la formation d'une seconde passe de seconds points d'entrée-sortie (406, 408, 414, 416, 506, 508, 514, 516, 616, 618, 628, 715) le long de la partie de bord (302, 304, 602, 708) qui alternent avec les premiers points d'entrée-sortie (406, 408, 414, 416, 506, 508, 514, 516, 616, 618, 628, 715) de telle sorte que des premier et second points d'entrée-sortie (406, 408, 414, 416, 506, 508, 514, 516, 616, 618, 628, 715) s'étendent par-delà des surfaces opposées de la collerette externe (106, 300, 652) entre chaque paire correspondante de deux ouvertures adjacentes.

4. Procédé selon l'une quelconque des revendications 1 à 3, la formation de la ligne de couture (402, 404, 502, 504, 612, 614) comprenant la formation de la pluralité de points d'entrée-sortie (406, 408, 414, 416, 506, 508, 514, 516, 616, 618, 628, 715) et de la pluralité de boucles (410, 510, 512, 528, 620) par :
l'extension d'une suture (412, 513, 526, 622, 636, 710, 712, 714), dans une direction qui est parallèle au bord libre, sur une première surface (322) de la collerette externe (106, 300, 652) et à travers la collerette externe (106, 300, 652) jusqu'à une seconde surface (324) de la collerette externe (106, 300, 652) qui est opposée à la première surface (322), formant ainsi un premier point d'entrée-sortie par-delà la première surface (322) ;
l'extension de la suture (412, 513, 526, 622, 636, 710, 712, 714) par-delà la seconde surface (324) vers le bord libre, sur le bord libre et en retour par-delà la première surface (322) jusqu'à une extrémité du premier point d'entrée-sortie, formant ainsi une première boucle (510) ; et
le passage de la suture (412, 513, 526, 622, 636, 710, 712, 714) en retour à travers la collerette externe (106, 300, 652) et par-delà la seconde surface (324) et à travers la collerette externe (106, 300, 652) jusqu'à la première surface (322), formant ainsi un second point d'entrée-sortie par-delà la seconde surface (324) et le procédé de formation de la ligne de couture (402, 404, 502, 504, 612, 614) étant répété de manière alternée par-delà les première et seconde surfaces (324) de la collerette externe (106, 300, 652) afin de former la pluralité de points d'entrée-sortie (406, 408, 414, 416, 506, 508, 514, 516, 616, 618, 628, 715) et la pluralité de boucles (410, 510, 512, 528, 620) de la ligne de couture (402, 404, 502, 504, 612, 614).

5. Procédé selon l'une quelconque des revendications 1 à 4, l'attache de la partie de bord (302, 304, 602, 708) aux entretoises (130, 132, 134, 136, 138, 140, 166, 168, 182, 184, 186, 422, 552, 562, 564, 646) par extension de la pluralité de points de surjet (420, 522, 560, 632) à travers la pluralité de boucles (410, 510, 512, 528, 620) et autour des entretoises (130, 132, 134, 136, 138, 140, 166, 168, 182, 184, 186, 422, 552, 562, 564, 646) comprenant l'extension d'au moins une partie (146, 148, 150, 156, 210, 224, 228, 302, 304, 602, 644, 708) des points de surjet (420, 522, 560, 632) autour de chaque entretoise (130, 132, 134, 136, 138, 140, 166, 168, 182, 184, 186, 422, 552, 562, 564, 646) à travers une boucle correspondante de la pluralité de boucles (410, 510, 512, 528, 620) et autour de la boucle correspondante de telle sorte qu'une double boucle (528) est formée autour de la boucle correspondante.

6. Procédé selon l'une quelconque des revendications 1 à 5, la partie de bord (302, 304, 602, 708) étant une partie de bord de sortie (306) disposée à l'opposé d'une partie de bord d'entrée (308) de la collerette externe (106, 300, 652), la rangée d'entretoises (130, 132, 134, 136, 138, 140, 166, 168, 182, 184, 186, 422, 552, 562, 564, 646) s'étendant circonférentiellement, à laquelle la partie de bord de sortie (306) est attachée, étant une première rangée d'entretoises (130, 132, 134, 136, 138, 140, 166, 168, 182, 184, 186, 422, 552, 562, 564, 646) s'étendant circonférentiellement du cadre (102, 550, 650) et le procédé comprenant en outre la fixation de la partie de bord d'entrée (308) de la collerette externe (106, 300, 652) à une deuxième rangée d'entretoises (130, 132, 134, 136, 138, 140, 166, 168, 182, 184, 186, 422, 552, 562, 564, 646) s'étendant circonférentiellement du cadre (102, 550, 650) qui définit une extrémité d'entrée (108, 648) du cadre (102, 550, 650) et qui est écartée axialement de la première rangée d'entretoises (130, 132, 134, 136, 138, 140, 166, 168, 182, 184, 186, 422, 552, 562, 564, 646) s'étendant circonférentiellement du cadre (102, 550, 650), de préférence, la première rangée d'entretoises (130, 132, 134, 136, 138, 140, 166, 168, 182, 184, 186, 422, 552, 562, 564, 646) s'étendant circonférentiellement du cadre (102, 550, 650) étant disposée de manière adjacente à une troisième rangée d'entretoises (130, 132, 134, 136, 138, 140, 166, 168, 182, 184, 186, 422, 552, 562, 564, 646) s'étendant circonférentiellement du cadre (102, 550, 650) qui définit une extrémité de sortie (110) du cadre (102, 550, 650).

7. Valvule cardiaque prothétique (100, 250), comprenant :
un cadre annulaire comprenant une pluralité d'entretoises (116) interconnectées agencées en une pluralité de rangées d'entretoises (130, 132, 134, 136, 138, 140, 166, 168, 182, 184, 186, 422, 552, 562, 564, 646) s'étendant circonférentiellement comprenant une première rangée d'entretoises (130, 132, 134, 136, 138, 140, 166, 168, 182, 184, 186, 422, 552, 562, 564, 646) s'étendant circonférentiellement qui est disposée plus près d'une extrémité de sortie (110) que d'une extrémité d'entrée (108, 648) du cadre (102, 550, 650) ;
une collerette externe (106, 300, 652) disposée autour d'une surface externe (172) du cadre (102, 550, 650) et comprenant une partie de bord de sortie (306) avec un bord de sortie libre, la partie de bord de sortie (306) s'étendant circonférentiellement autour du cadre (102, 550, 650) et comprenant une ligne de couture (402, 404, 502, 504, 612, 614) comprenant :
une pluralité de points d'entrée-sortie (406, 408, 414, 416, 506, 508, 514, 516, 616, 618, 628, 715) s'étendant le long de la partie de bord de sortie (306) parallèlement au bord de sortie (320, 604) mais écartés de celui-ci ; et
une pluralité de points en boucle qui sont espacés les uns des autres dans une direction de la pluralité de points d'entrée-sortie (406, 408, 414, 416, 506, 508, 514, 516, 616, 618, 628, 715), chaque point en boucle (410, 510, 512, 528, 620) tournant autour du bord de sortie (320, 604) et s'étendant entre le bord de sortie (320, 604) et une intersection de deux points d'entrée-sortie (406, 408, 414, 416, 506, 508, 514, 516, 616, 618, 628, 715) adjacents correspondants de la pluralité de points d'entrée-sortie (406, 408, 414, 416, 506, 508, 514, 516, 616, 618, 628, 715) ; et
une pluralité de points de surjet (420, 522, 560, 632) s'étendant à travers la pluralité de points en boucle et autour d'entretoises (130, 132, 134, 136, 138, 140, 166, 168, 182, 184, 186, 422, 552, 562, 564, 646) de la première rangée d'entretoises (130, 132, 134, 136, 138, 140, 166, 168, 182, 184, 186, 422, 552, 562, 564, 646) s'étendant circonférentiellement de telle sorte que le bord de sortie (320, 604) est attaché aux entretoises (130, 132, 134, 136, 138, 140, 166, 168, 182, 184, 186, 422, 552, 562, 564, 646), contre des surfaces orientées radialement vers l'extérieur des entretoises (130, 132, 134, 136, 138, 140, 166, 168, 182, 184, 186, 422, 552, 562, 564, 646), de préférence
le bord de sortie (320, 604) étant plus rugueux qu'une partie restante de la partie de bord de sortie (306) de la collerette externe (106, 300, 652) et/ou
la valvule cardiaque comprenant en outre une pluralité de feuillets (112, 600, 702) fixés à l'intérieur du cadre (102, 550, 650) et la pluralité de feuillets (112, 600, 702) faisant face à des surfaces orientées radialement vers l'intérieur des entretoises (130, 132, 134, 136, 138, 140, 166, 168, 182, 184, 186, 422, 552, 562, 564, 646).

8. Valvule cardiaque prothétique (100, 250) selon la revendication 7, une pluralité de rangées d'entretoises (130, 132, 134, 136, 138, 140, 166, 168, 182, 184, 186, 422, 552, 562, 564, 646) s'étendant circonférentiellement comprenant en outre une deuxième rangée d'entretoises (130, 132, 134, 136, 138, 140, 166, 168, 182, 184, 186, 422, 552, 562, 564, 646) s'étendant circonférentiellement qui définit l'extrémité de sortie (110) du cadre (102, 550, 650) et la première rangée d'entretoises (130, 132, 134, 136, 138, 140, 166, 168, 182, 184, 186, 422, 552, 562, 564, 646) s'étendant circonférentiellement étant disposée de manière adjacente à la deuxième rangée d'entretoises (130, 132, 134, 136, 138, 140, 166, 168, 182, 184, 186, 422, 552, 562, 564, 646) s'étendant circonférentiellement.

9. Valvule cardiaque prothétique (100, 250) selon l'une quelconque des revendications 7 ou 8, la collerette externe (106, 300, 652) comprenant une rangée d'ouvertures espacées s'étendant le long de la partie de bord de sortie (306) parallèlement au bord de sortie (320, 604) mais écartées de celui-ci et la pluralité de points d'entrée-sortie (406, 408, 414, 416, 506, 508, 514, 516, 616, 618, 628, 715) et la pluralité de points en boucle s'étendant à travers les ouvertures espacées de la rangée d'ouvertures espacées.

10. Valvule cardiaque prothétique (100, 250) selon l'une quelconque des revendications 7 à 9, la pluralité de points d'entrée-sortie (406, 408, 414, 416, 506, 508, 514, 516, 616, 618, 628, 715) étant écartés du bord de sortie (320, 604) d'une distance (418, 518, 630) qui s'étend perpendiculairement au bord de sortie (320, 604), de préférence la distance (418, 518, 630) étant située dans une plage de 0,5 à 2,0 mm.

11. Valvule cardiaque prothétique (100, 250) selon l'une quelconque des revendications 7 à 10, la partie de bord de sortie (306) comprenant une pluralité de saillies (312) qui définissent une forme ondulée de la partie de bord de sortie (306) qui suit un contour de la première rangée d'entretoises (130, 132, 134, 136, 138, 140, 166, 168, 182, 184, 186, 422, 552, 562, 564, 646) s'étendant circonférentiellement.

12. Procédé comprenant :
la formation d'une ligne de couture (402, 404, 502, 504, 612, 614) le long d'une partie (602, 708) de bord de cuspide d'un feuillet (112, 600, 702) pour une valvule cardiaque prothétique (100, 250) présentant une pluralité de points d'entrée-sortie (406, 408, 414, 416, 506, 508, 514, 516, 616, 618, 628, 715) qui s'étendent le long de la partie (602, 708) de bord de cuspide, écartés d'un bord (608) de cuspide le plus à l'extérieur de la partie (602, 708) de bord de cuspide et une pluralité de boucles (410, 510, 512, 528, 620) qui sont espacées les unes des autres dans une direction des points d'entrée-sortie (406, 408, 414, 416, 506, 508, 514, 516, 616, 618, 628, 715), chaque boucle tournant autour du bord (608) de cuspide de la partie (602, 708) de bord de cuspide et s'étendant entre le bord (608) de cuspide et une intersection de deux points d'entrée-sortie (406, 408, 414, 416, 506, 508, 514, 516, 616, 618, 628, 715) adjacents ; et
l'attache de la partie (602, 708) de bord de cuspide à un composant cible (634) de la valvule cardiaque prothétique (100, 250) par extension d'une pluralité de points de surjet (420, 522, 560, 632) à travers la pluralité de boucles (410, 510, 512, 528, 620) et autour du composant cible (634) ou à travers celui-ci de telle sorte que le bord (608) de cuspide de la partie (602, 708) de bord de cuspide est placé contre le composant cible (634).

13. Procédé selon la revendication 12, le composant cible (634) étant une collerette en tissu conçue pour être attachée à une surface interne d'un cadre annulaire de la valvule cardiaque prothétique (100, 250).

14. Procédé selon la revendication 12, le composant cible (634) étant des entretoises (130, 132, 134, 136, 138, 140, 166, 168, 182, 184, 186, 422, 552, 562, 564, 646) d'un cadre annulaire de la valvule cardiaque prothétique (100, 250) qui suivent une ligne échancrée de la partie (602, 708) de bord de cuspide.

15. Procédé selon l'une quelconque des revendications 12 à 14, la pluralité de points d'entrée-sortie (406, 408, 414, 416, 506, 508, 514, 516, 616, 618, 628, 715) étant une première passe de premiers points d'entrée-sortie (406, 408, 414, 416, 506, 508, 514, 516, 616, 618, 628, 715) formés le long de la partie (602, 708) de bord de cuspide et la formation de la ligne de couture (402, 404, 502, 504, 612, 614) comprenant en outre la formation d'une seconde passe de seconds points d'entrée-sortie (406, 408, 414, 416, 506, 508, 514, 516, 616, 618, 628, 715) le long de la partie (602, 708) de bord de cuspide qui alternent avec les premiers points d'entrée-sortie (406, 408, 414, 416, 506, 508, 514, 516, 616, 618, 628, 715) de telle sorte que des premier et second points d'entrée-sortie (406, 408, 414, 416, 506, 508, 514, 516, 616, 618, 628, 715) s'étendent par-delà des côtés opposés du feuillet (112, 600, 702) entre chaque paire de boucles (410, 510, 512, 528, 620) adjacentes de la pluralité de boucles (410, 510, 512, 528, 620).
